# EUROPEAN PATENT APPLICATION

(11) **EP 2 269 560 A1**
(43) Date of publication of application: **05.01.2011**
(21) Application number: 10758221.5
(22) Date of filing: 25.03.2010
(51) Int. Cl.: A61J 3/00, G06K 17/00, G06Q 50/00

(54) **MEDICINE MANAGEMENT METHOD**

(30) Priority: 31.03.2009 JP 2009084545; 23.07.2009 JP 2009171702
(71) Applicant: Panasonic Corporation, Kadoma-shi Osaka 571-8501 (JP)
(72) Inventor: GOTOU, Makoto, Osaka-shi, Osaka 540-6207 (JP); MATSUKAWA, Yoshihiko, Osaka-shi, Osaka 540-6207 (JP); TANIMOTO, Takanobu, Osaka-shi, Osaka 540-6207 (JP); NAKAMURA, Tohru, Osaka-shi, Osaka 540-6207 (JP); TAKAHASHI, Rie, Osaka-shi, osaka 540-6207 (JP)
(74) Representative: Kügele, Bernhard
(86) International application number: PCT/JP2010/002107
(87) International publication number: WO 2010/113436

(57) **Abstract**

A medicine management method includes: a prescription generating step of generating a prescription direction; and a dispensation operation step of making dispensation information regarding dispensation correspond to a medicine container and a prescription code, to update management information of a dispensation managing section. The medicine management method includes a withdrawal operation step of specifying the medicine container that corresponds to a prescription code cancelled in accordance with the management information at the time of a cancellation of the prescription direction in the dispensation operation step, to direct a withdrawal process on the medicine container based upon cancellation information. An accurate medicine withdrawal is performed on the medicine container cancelled.

## Description

### TECHNICAL FIELD

The present invention relates to a medicine management method in a medicine dispensation operation where a dispensation operator such as a nurse gets and prepares medicines.

### BACKGROUND ART

As a conventional dispensation support, there has been a medicine setting system for supporting procurement of medicines corresponding to a prescription (e.g. refer to Patent Document 1). FIG. 32 is a schematic constitutional view of the medicine setting system. FIG. 33 is a display example of an information processor in a medicine setting system of Patent Document 1.

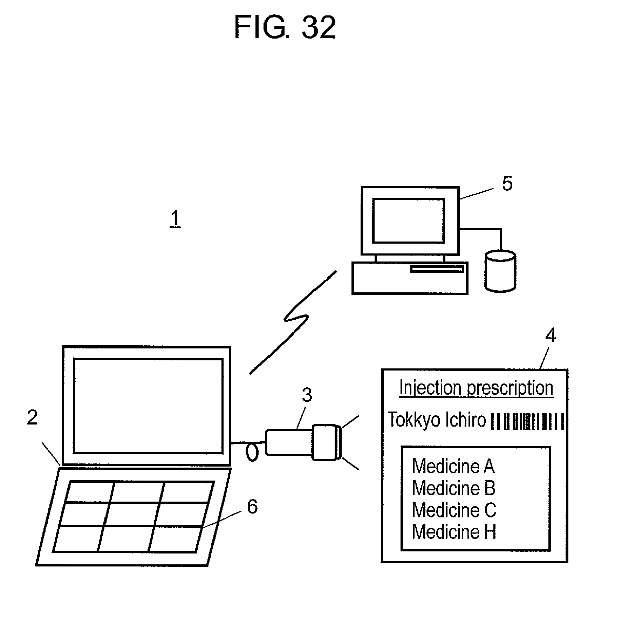

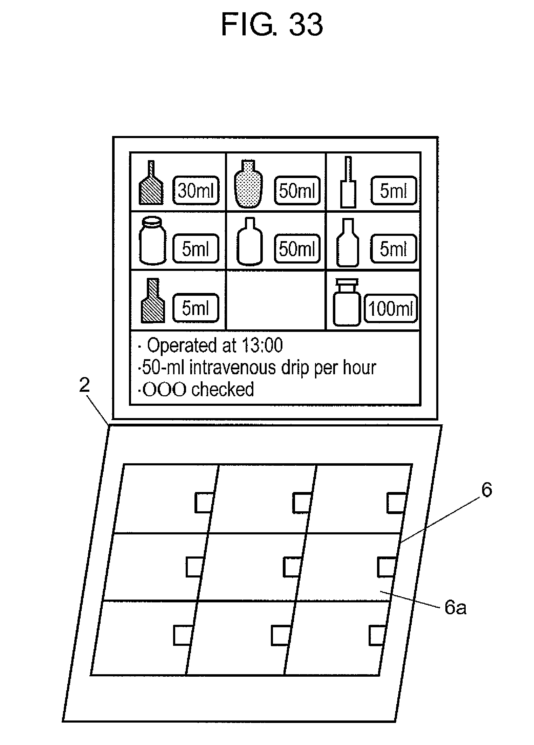

In FIG. 32, an information processor 2 in a medicine setting system 1 has a bar code reader 3. First, a bar code of an injection prescription 4 is read for performing dispensation. Based upon the read bar code information, the information processor 2 inquires of a server 5 about medicine information. The server 5 receives the bar code information, and searches a medicine information management table. The server 5 then transmits medicine information of the injection prescription as a search result to the information processor 2. As shown in FIG. 33, the information processor 2 displays the received medicine information in divided windows. A special medicine tray 6 installed previously is sectioned into nine regions 6a. On the screen of the information processor 2, the received medicine information is displayed in nine regions 6a divided in correspondence to that sectioning.

In the above situation, a dispensation operator such as a nurse brings corresponding medicines (not shown) while viewing the display of the medicine information on the screen of the information processor 2. The operator then sets the medicines in the regions of the medicine tray 6 which correspond to the screen regions of the information processor 2.

In such a manner, an accurate dispensation operation is performed by use of the medicine setting system 1. However, in a hospital, there often occurs a modification or cancellation of an issued prescription in the process of dispensation based upon the prescription.

When such a modification or cancellation of a prescription occurs, a medicine withdrawing operation is performed so as to reuse the cancelled medicine. The dispensation operator needs to manually perform the withdrawal operation from the cancelled prescription. In this case, the dispensation operator looks for the medicine tray corresponding to the cancelled prescription. This operation is laborious since being performed by a human operator. Further, it may cause an error.

Moreover, in determining whether or not a medicine is a reusable withdrawal medicine from the cancelled prescription, the dispensation operator may make an error of determining a reusable withdrawal medicine as a disposal medicine. For example, a requiring cool medicine that needs to be kept cool taken out of a cool box is not reusable and becomes a disposal medicine when being in a normal-temperature environment for a certain period of time. The dispensation operator can make an accurate determination on a withdrawal medicine when knowing a normal-temperature operation time for the requiring cool medicine being in the normal-temperature environment after taken out of the cool box.

However, in the conventional medicine management system such as the medicine setting system 1, dispensation operation information has not been sufficiently managed, and hence the dispensation operator does not know management information such as the normal-temperature operation time for the medicine, and may determine even a withdrawal medicine as a disposal medicine in view of the safety. This means that the reusable withdrawal medicine is treated as the disposal medicine, and this leads the hospital to incur an economic loss.

There has been a problem with the use of the conventional medicine management system in performing the withdrawal operation, since dispensation operation information has not been sufficiently managed with respect to each prescription, namely, with respect to each patient, an accurate withdrawal operation cannot be performed.

### PRIOR ART DOCUMENT

### PATENT DOCUMENT

[Patent Document 1] Unexamined Japanese Patent Publication No. 2004-70803

### DISCLOSURE OF THE INVENTION

The present invention is to solve this problem, and provide a medicine management method for accurately specifying a medicine container, cancellation of which has occurred, and sufficiently managing information in performing a withdrawal operation.

A medicine management method of the present invention is a medicine management method, including: a prescription generating step of generating a prescription direction, which includes a prescription code and dispensation direction information, in a prescription generating section; and a dispensation operation step of communicating with a dispensation managing section, by sending dispensation information regarding each medicine container dispensed based upon the prescription direction, while making the dispensation information correspond to the medicine container and the prescription code, and updating management information of the dispensation managing section, wherein the method includes a withdrawal operation step of specifying the medicine container that corresponds to a prescription code cancelled in accordance with the management information at the time of cancellation of the prescription direction in the dispensation operation step, and directing withdrawal process on the medicine container based upon cancellation information.

According to this method, it is possible to specify a cancelled medicine container and perform accurately a withdrawal process, so as to reduce medicine withdrawal errors.

### BRIEF DESCRIPTION OF THE DRAWINGS

FIG. 1 is a flowchart for a medicine management method in an embodiment 1 of the present invention.
FIG. 2 is a conceptual view of a medicine management system in the embodiment 1.
FIG. 3 is a flowchart for medicine management, with a dispensation operation step divided into a plurality of steps, in the embodiment 1.
FIG. 4 is a block diagram of the medicine management system in the embodiment 1.
FIG. 5 is a flowchart for an occurrence of prescription in a prescription generating section in the embodiment 1.
FIG. 6 is a schematic constitutional view of a medicine supplying section in the embodiment 1.
FIG. 7 is a view for explaining a medicine supply of the medicine supplying section in the embodiment 1.
FIG. 8 is a flowchart for a medicine supply of the medicine supplying section in the embodiment 1.
FIG. 9 is a schematic constitutional view of a medicine procuring section in the embodiment 1.
FIG. 10 is a view showing a display screen of a procurement display of the medicine procuring section in the embodiment 1.
FIG. 11 is a view showing a brief overview of a container correspondence display of the medicine procuring section in the embodiment 1.
FIG. 12 is a flowchart for a medicine procurement and a procurement inspection in the medicine procuring section in the embodiment 1.
FIG. 13 is a schematic constitutional view of a medicine mixing section in the embodiment 1.
FIG. 14 is a flowchart for a medicine mixture and a mixture inspection in the medicine mixing section in the embodiment 1.
FIG. 15 is a schematic constitutional view of a medicine delivering section in the embodiment 1.
FIG. 16 is a flowchart for a delivery operation of the medicine delivering section in the embodiment 1.
FIG. 17 is a flowchart for a progress inquiry in a progress inquiring section in the embodiment 1.
FIG. 18 is a flowchart for updating information of a medicine managing section in the embodiment 1.
FIG. 19 is a schematic constitutional view of a medicine withdrawing section in the embodiment 1.
FIG. 20 is a flowchart showing a brief overview of a withdrawal operation of the medicine withdrawing section in the embodiment 1.
FIG. 21 is the first flowchart for a withdrawal operation of the medicine withdrawing section in the embodiment 1.
FIG. 22 is the second flowchart for the withdrawal operation of the medicine withdrawing section in the embodiment 1.
FIG. 23 is the third flowchart for the withdrawal operation of the medicine withdrawing section in the embodiment 1.
FIG. 24 is the fourth flowchart for the withdrawal operation of the medicine withdrawing section in the embodiment 1.
FIG. 25 is the fifth flowchart for the withdrawal operation of the medicine withdrawing section in the embodiment 1.
FIG. 26 is the sixth flowchart for the withdrawal operation of the medicine withdrawing section in the embodiment 1.
FIG. 27 is the seventh flowchart for the withdrawal operation of the medicine withdrawing section in the embodiment 1.
FIG. 28 is a diagram showing a medicine management table in the embodiment 1.
FIG. 29 is a front view of a cancelled medicine in the embodiment 1.
FIG. 30 is a diagram showing a cancelled medicine management table in the embodiment 1.
FIG. 31 is a flowchart for a return operation of the medicine procuring section in the embodiment 1.
FIG. 32 is a schematic constitutional view of a conventional medicine setting system.
FIG. 33 is a display example of an information processor in the conventional medicine setting system.

### DESCRIPTION OF EMBODIMENTS

Hereinafter, an embodiment of the present invention is described with reference to drawings. It is to be noted that in the following descriptions, the same constitutional components are provided with the same reference marks, and hence the descriptions thereof may be omitted.

### Embodiment 1

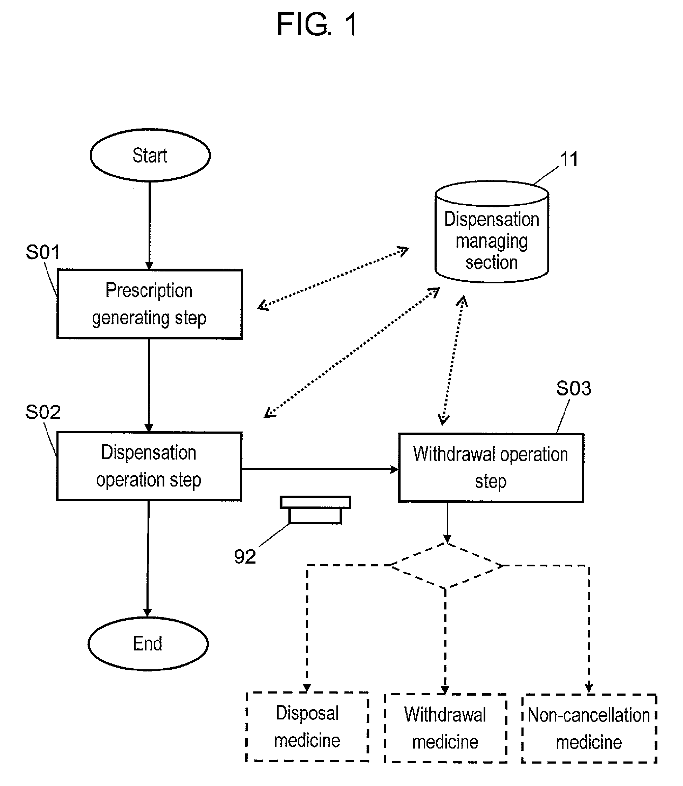

FIG. 1 is a flowchart for a medicine management method in the embodiment 1 of the present invention. The medicine management method of the embodiment 1 has a prescription generating step S01 and a dispensation operation step S02. Further, the medicine management method has a withdrawal operation step S03 of directing a withdrawal process on a medicine container 92 at the time of cancellation of a prescription direction in the dispensation operation step S02. It is to be noted that the medicine container 92 is a container for storing and delivering medicines.

The prescription generating step S01 generates a prescription direction, which includes a prescription code and dispensation direction information, in a prescription generating section.

The dispensation operation step S02 dispenses medicines to each medicine container 92 based upon each prescription direction, respectively. Dispensation information in regard to the dispensation is then communicated to a dispensation managing section 11, in relation to the medicine container 92 and the prescription code of the prescription direction, so as to update management information of the dispensation managing section 11.

When a prescription direction is cancelled in the dispensation operation step S02, the withdrawal operation step S03 obtains a cancelled prescription code and cancellation information. The medicine container 92 corresponding to the cancelled prescription code is then specified by the use of the management information. A withdrawal process on the cancelled medicine container 92 specified is directed, based upon the cancellation information.

In this medicine management method, the cancelled prescription code, the cancellation information and the medicine container are linked. In the case of performing the withdrawal process on the cancelled medicine container 92, the withdrawal process on the medicine container 92 is performed, based upon the cancellation information.

It should be noted that the cancellation information means contents of a cancellation direction to a prescription, and includes a name of a cancelled medicine and the number thereof.

The withdrawal operation step S03, for example, determines, in the withdrawal process, whether or not a medicine in the cancelled medicine container 92 corresponds to a withdrawal medicine, based upon the management information and the cancellation information of the medicine container 92.

For example, the management information includes information of the storage medicines, which indicates medicine names stored in the medicine container 92 and the number of each medicine name. This storage medicine information is compared with the cancellation information, so as to determine from the medicine name whether or not a cancelled medicine in the medicine container 92 is a withdrawal medicine. Then, an accurate determination is possible even in a situation where all medicines in the prescription have not been procured in the medicine container due to the dispensation operation in progress.

There further is a medicine, regarding which accurate determination as to whether or not the medicine corresponds to a reusable withdrawal medicine cannot be made only in accordance with its medicine name and the cancellation information. With use of dispensation progress information in the management information, it is possible to determine whether or not the cancelled medicine is reusable. For example, when a mixture medicine has already been mixed, the mixed medicine is determined not as a withdrawal medicine but as a disposal medicine. For example, when a requiring cool medicine that needs to be kept cool is in the normal-temperature environment for a long period of time, the requiring cool medicine is determined not as a withdrawal medicine but as a disposal medicine.

Moreover, the withdrawal operation step S03, for example, shows the cancellation information on the cancelled medicine container 92, as a withdrawal process. An ID code of the cancelled medicine container 92 is obtained in a place where the withdrawal operation is performed, and the cancellation information linked to this ID code of the medicine container 92 is showed. This makes a withdrawal person know the cancellation information of the cancelled medicine container 92, thereby to accurately withdraw a medicine.

Furthermore, the withdrawal operation step S03, for example, determines, as the withdrawal process, whether each medicine in the cancelled medicine container 92 corresponds to a withdrawal medicine, corresponds to a disposal medicine, or corresponds to a non-cancelled medicine, based upon the management information and the cancellation information of the medicine container.

In the case of occurring a cancellation, it is possible to classify at once each medicine held in the medicine container 92 to one among a withdrawal medicine, a disposal medicine and a non-cancelled medicine.

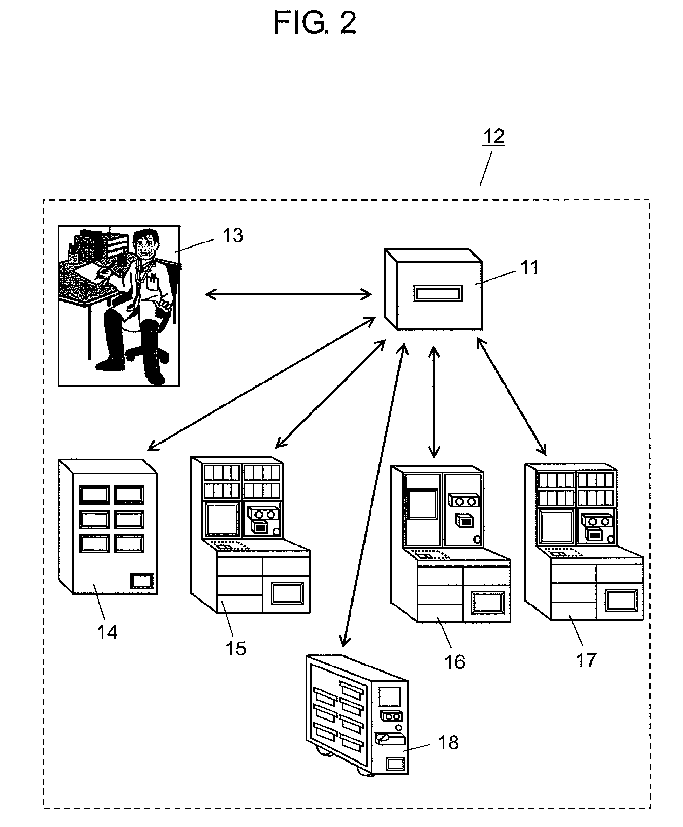

Next, the medicine management system in the embodiment 1 is described in detail. FIG. 2 is a conceptual view of the medicine management system in the embodiment 1. It is to be noted that in the following drawings, a solid line indicates that information is mutually exchangeable.

As shown in FIG. 2, the medicine management system 12 of the embodiment 1 has a prescription generating section (not shown), a progress inquiring section (not shown), a medicine supplying section 14, a medicine procuring section 15, a medicine mixing section 16, a medicine withdrawing section 17, a medicine delivering section 18, and a dispensation managing section 11.

The prescription generating section is an apparatus, located in a medical office 13, for a doctor making a prescription and generating a prescription direction to a patient. The progress inquiring section is an apparatus, also located in the medical office 13, for inquiring a progress status and clarifying the progress status. The medicine supplying section 14 automatically supplies a medicine based upon a prescription. The medicine procuring section 15 is an apparatus for replenishing and procuring a medicine that cannot be automatically supplied in the medicine supplying section 14. The medicine mixing section 16 is an apparatus for mixing at least either a medicine supplied in the medicine supplying section 14 or a medicine procured in the medicine procuring section 15. The medicine withdrawing section 17 is an apparatus for withdrawing a medicine subjected to the dispensation operation in the case of a cancellation of the prescription, or in some other case. The medicine delivering section 18 is an apparatus for delivering medicines among the apparatuses in the medicine management system 12. The dispensation managing section 11 is an apparatus for managing information among these apparatuses.

Further, the medicine management system 12 uses a medicine container 92 for performing a medicine dispensation operation. Specifically, a medicine tray is used. This medicine container 92 is equipped with an RFID tag having a specific ID code. In other words, the medicine container 92 is equipped with an identification tag element having an identification code. The medicine management system 12 uses the medicine container 92 in the entire system. One medicine container 92 moves to one among the medicine supplying section 14, the medicine procuring section 15, the medicine mixing section 16, the medicine withdrawing section 17 and the medicine delivering section 18. A variety of the dispensation operations are performed on the medicine container 92. It is to be noted that the medicine container 92 may be a medicine storage container that stores and delivers medicines, and may be a medicine bag for delivery or a box for delivery, for example.

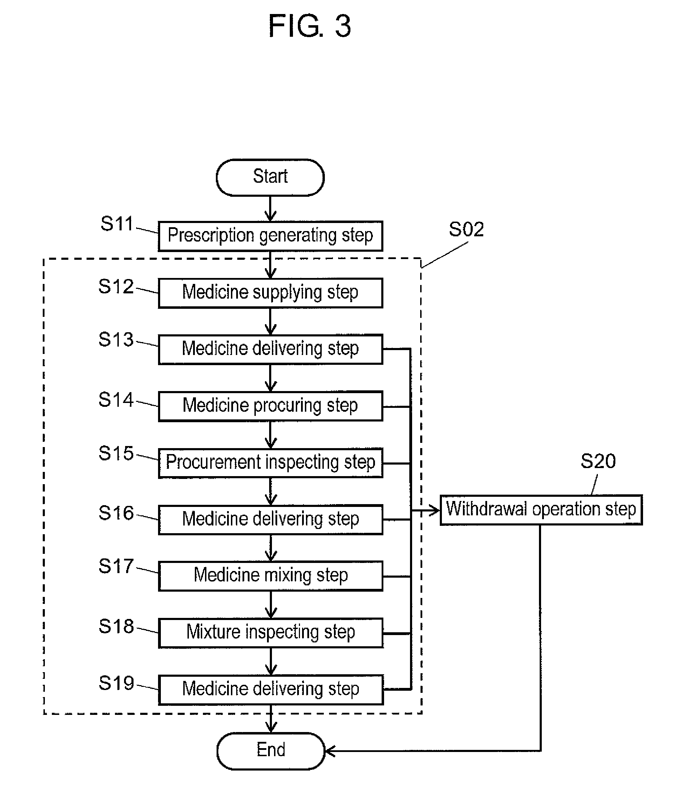

A series of flow of the medicine management system 12 as thus configured is described with the reference to FIG. 3. FIG. 3 is a flowchart for a medicine management with a dispensation operation step divided into a plurality of steps.

In FIG. 3, first, a prescription generating step S11 is performed for generating prescription in the prescription generating section. Next, a medicine supplying step S12 is performed for automatically supplying a medicine based upon the prescription generated in the medicine supplying section 14. A medicine delivering step S13 is then performed for delivering the medicine container 92, inserted with the supplied medicines, by means of the medicine delivering section 18 or the like.

Subsequently, a medicine procuring step S14 is performed for manually replenishing and procuring a medicine into the medicine container 92 delivered to the medicine procuring section 15. A procurement inspecting step S15 is then performed for inspecting whether or not the replenishment and procurement have been completed with accuracy. A medicine delivering step S16 is performed for delivering the medicine container 92 in the state of having completed the replenishment and procurement without a problem, by means of the medicine delivering section 18 or the like.

Thereafter, a medicine mixing step S17 is performed for taking required medicines out of the medicine container 92 delivered to the medicine mixing section 16, so as to mix the required medicines in the medicine mixing section 16. A mixture inspecting step S18 is performed for inspecting whether or not the medicine mixture has been accurately completed. A medicine delivering step S19 is then performed for delivering the medicine container 92, inserted with the medicine in the state of having completed the mixture without a problem, by means of the medicine delivering section 18 to deliver the medicines to a patient.

It should be noted that the dispensation operation step S02 shown in FIG. 1 means a plurality of dispensation operations performed in a plurality of dispensation operating sections. The plurality of dispensation operations in steps S12 to S19 are examples of the step S02.

Specifically, the dispensation operation step S02 has at least one among the medicine supplying step S12, the medicine delivering step S13, the medicine procuring step S14, the procurement inspecting step S15, the medicine delivering step S16, the medicine mixing step S17, the mixture inspecting step S18, and the medicine delivering step S19.

The medicine supplying step S12 automatically supplies medicine in accordance with the prescription direction in the medicines withdrawing section 17 and inserts the medicine into the medicine container 92. The ID code of the medicine container 92 and the prescription code of the prescription direction are made to have a corresponding relation to each other. The corresponding relation and supply information regarding the medicines inserted into the medicine container 92 are communicated to the dispensation managing section 11, so as to update management information of the dispensation managing section 11. In addition, the supply information indicating contents of the medicine supply is one piece of the dispensation information. Further, updating the management information implies storing the updated management information into the dispensation managing section 11.

In the medicine procuring section 15, the medicine procuring step S14 checks an authenticated procurement person by comparing with register information. The ID code of the medicine container 92 is identified, and supplementary medicine information on the medicine container 92 is showed, based upon the management information of the dispensation managing section 11 corresponding to the ID code of the medicine container 92. Procurement information, including procurement person information, is communicated to the dispensation managing section 11, while being made to correspond to the ID code of the medicine container 92, to update the management information. In addition, the procurement information indicating contents of the medicine procurement is one piece of the dispensation information.

In the medicine procuring section 15, the procurement inspecting step S15 checks an authenticated procurement inspector by comparing with register information. Based upon the management information of the dispensation managing section 11 corresponding to the ID code of the medicine container 92, procurement medicine information procured in the medicine container 92 is showed. Procurement inspection information, including procurement inspector information, is communicated to dispensation managing section 11, while being made to correspond to the ID code of medicine container 92, to update the management information. The procurement inspection information indicating contents of procurement inspection is one piece of the dispensation information.

In the medicine mixing section 16, the medicine mixing step S17 authenticates a mixing person and makes a check by comparing with register information. The ID code of the medicine container 92 is identified, and mixture inspection medicine information on the mixture medicine of the medicine container 92 is showed, based upon the management information of dispensation managing section 11 which corresponds to the ID code of medicine container 92. Mixture information, including mixing person information, is communicated to dispensation managing section 11, while being made to correspond to the ID code of the medicine container 92, to update the management information. In addition, the mixture information indicating contents of medicine mixture is one piece of the dispensation information.

In the medicine mixing section 16, the mixture inspecting step S18 checks an authenticated mixing inspector by comparing with register information. Based upon the management information of the dispensation managing section 11 corresponding to the ID code of the medicine container 92, mixture medicine information on the medicine container 92 is showed. Mixture inspection information, including mixing inspector information, is communicated to the dispensation managing section 11, while being made to correspond to the ID code of the medicine container 92, to update the management information. In addition, the mixture inspection information indicating contents of mixture inspection is one piece of the dispensation information.

The dispensation operation step S02 can manage dispensation information and dispensation progress with respect to each medicine container 92 even when a plurality of dispensations are performed. In addition, each of the supply information, the procurement information, the procurement inspection information, the mixture information and the mixture inspection information is a part of the dispensation information.

Further, when a prescription is cancelled from a medical office or the like where the progress inquiring section and the prescription generating section are located, the medicine container 92 in the dispensation operation of steps S12 to S19 at the time is moved to a medicine withdrawing section 17. Medicines are then withdrawn in an operation step S20. It is to be noted that the medicines withdrawn in the step S20 can be returned to the medicine supplying section 14, the medicine procuring section 15 or the medicine mixing section 16 by managing the number of withdrawn medicines in the dispensation managing section 11 or the like.

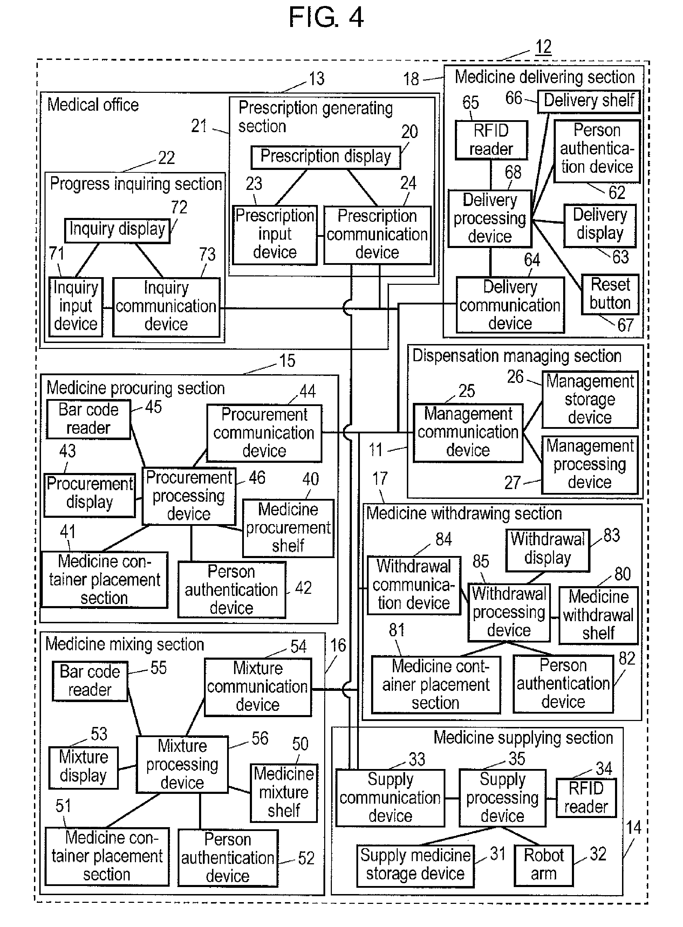

Subsequently, details of respective configurations of the medicine management system 12 are described. FIG. 4 shows a block diagram of the medicine management system 12. A prescription generating section 21 and a progress inquiring section 22 are located in the medical office 13. The prescription generating section 21 generates a prescription direction including a prescription code and dispensation direction information based upon prescription given by a doctor to a patient. The progress inquiring section 22 inquires a progress status of the dispensation with respect to the prescription direction.

It should be noted that the case is described in the embodiment 1 where a prescription generating section 21 and a progress inquiring section 22 are located in the medical office 13. However, so long as the operation of the system is considered, these configurations, at least either the prescription generating section 21 or the progress inquiring section 22, can also be appropriately arranged in another place such as a pharmacy, a medicine room, or a central control room.

The prescription generating section 21 has a prescription input device 23, a prescription communication device 24, and a prescription display 20. The prescription input device 23 has a keyboard, a touch panel and the like of a PC for inputting a prescription direction. It should be noted that the prescription direction includes a prescription code and dispensation direction information. The prescription communication device 24 communicates with a supply communication device of the medicine supplying section 14 and a management communication device of the dispensation managing section 11. The prescription display 20 displays a prescription direction inputted in the prescription input device 23. The dispensation direction information at this time is information on a dispensation to the patient based upon a prescription of the doctor, and is information related to a dispensation, such as a name of a medicine, the number thereof, and contents of a mixing process thereon. It is to be noted that the number of a medicine is used implying a quantity thereof, and the medicine is used implying a drug.

The dispensation managing section 11 is configured of an information management device, such as a server. The dispensation managing section 11 has a management communication device 25, a management storage device 26 and a management processing device 27. The management communication device 25 transmits and receives information to and from other devices. The management storage device 26 stores medicine management information. The management processing device 27 is a CPU or the like for performing information processing.

The medicine supplying section 14 automatically supplies a medicine in accordance with the prescription direction and inserts the medicine into the medicine container 92. Further, supply information regarding the medicine inserted into the medicine container 92 is communicated to the dispensation managing section 11, to update the management information.

To that end, the medicine supplying section 14 has supply a medicine storage device 31, a robot arm 32, a supply communication device 33, an RFID reader 34, and a supply processing device 35. The supply medicine storage device 31 stores supply medicines. The robot arm 32 carries a medicine from the supply medicine storage device 31 to the medicine container 92. The supply communication device 33 communicates with each apparatus of the medicine management system 12. The RFID reader 34 reads an RFID tag of the medicine container 92. The supply processing device 35 manages each device of the medicine supplying section 14.

The medicine procuring section 15 compares the medicine container 92 taken out of the medicine supplying section 14 with the dispensation direction information, and replenishes a medicine lacking in the medicine container 92. The medicine procuring section 15 performs medicine procurement and procurement inspection.

To that end, the medicine procuring section 15 has a medicine procurement shelf 40, a medicine container placement section 41, a person authentication device 42, a procurement display 43, a procurement communication device 44, a bar code reader 45, and a procurement processing device 46. The medicine procurement shelf 40 holds medicines therein. The medicine container placement section 41 is a placement section for placing the medicine container 92 thereon. The person authentication device 42 authenticates a procurement person and a procurement inspector. The procurement display 43 shows a variety of information. The procurement communication device 44 communicates information with each apparatus of the medicine management system 12. The bar code reader 45 reads medicine information on a medicine. The procurement processing device 46 controls each device of the medicine procuring section 15.

In the medicine mixing section 16, a medicine mixture, which is to mix medicines collected in the medicine container 92 as necessary, and a mixture inspection, which is to inspect the medicine mixture, are performed.

To that end, the medicine mixing section 16 has a medicine mixture shelf 50, a medicine container placement section 51, a person authentication device 52, a mixture display 53, a mixture communication device 54, a bar code reader 55, and a mixture processing device 56.

The medicine container placement section 51 is a placement section for placing the medicine container 92 thereon. The person authentication device 52 authenticates a mixing person and a mixing inspector. The mixture display 53 shows a variety of information. The mixture communication device 54 communicates information with each apparatus of the medicine management system 12. The bar code reader 55 reads medicine information on a medicine. The mixture processing device 56 controls each device of the medicine mixing section 16.

The medicine delivering section 18 delivers the medicine container 92 among the apparatuses of the medicine management system 12. To that end, the medicine delivering section 18 has a person authentication device 62, a delivery display 63, a delivery communication device 64, an RFID reader 65, a delivery shelf 66, a reset button 67, and a delivery processing device 68. The person authentication device 62 authenticates a delivery person. The delivery display 63 shows a variety of information. The delivery communication device 64 communicates information with each apparatus of the medicine management system 12. The RFID reader 65 detects the medicine container 92. The delivery shelf 66 holds the medicine container 92 therein. The reset button 67 releases a stopped state of the medicine delivering section 18. The delivery processing device 68 controls each device of the medicine delivering section 18.

The progress inquiring section 22 is an apparatus for a doctor, a nurse and a pharmacist to inquire about a progress status of a dispensation operation.

The progress inquiring operation is to show dispensation progress information corresponding to the prescription code in the progress inquiring section 22, based upon the management information of the dispensation managing section 11 corresponding to the prescription code.

To that end, the progress inquiring section 22 located in the medical office 13 has an inquiry input device 71, an inquiry display 72 and an inquiry communication device 73. The inquiry input device 71 is inputted with dispensation inquiry information including the prescription code. The inquiry display 72 displays dispensation progress information. The inquiry communication device 73 communicates information with each apparatus of the medicine management system 12.

The medicine withdrawing section 17 is an apparatus for performing an operation to withdraw a cancelled medicine in the case of occurring a cancellation of the whole or part of a prescription direction.

The medicine withdrawing operation is to specify the ID code of the medicine container 92 corresponding to the prescription code of the cancelled prescription direction in the medicine withdrawing section 17 when the cancellation of the prescription direction occurs. The operation is to then determine whether or not a medicine in the medicine container 92 corresponds to a withdrawal medicine based upon the cancellation information and the management information on the medicine container 92.

To that end, the medicine withdrawing section 17 has a medicine withdrawal shelf 80, a medicine container placement section 81, a person authentication device 82, a withdrawal display 83, a withdrawal communication device 84, and a withdrawal processing device 85. The medicine withdrawal shelf 80 holds medicines therein. The medicine container placement section 81 is the placement section for placing the medicine container 92 thereon. The person authentication device 82 authenticates a withdrawal person. The withdrawal display 83 shows a variety of information. The withdrawal communication device 84 communicates information with each apparatus of the medicine management system 12. The withdrawal processing device 85 controls each device of the medicine withdrawing section 17.

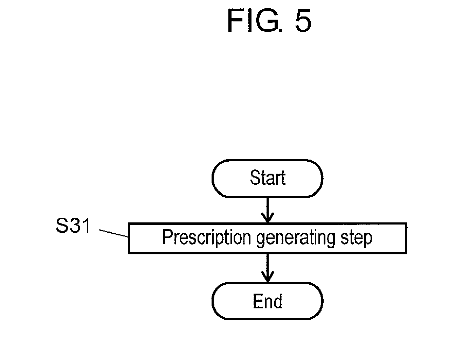

Next, an operation of each apparatus is described. FIG. 5 shows a flowchart for generating prescription in the prescription generating section 21 in the embodiment 1. In the prescription generating section 21, the prescription generating step S31 is performed for generating a prescription direction including a prescription code and dispensation direction information inputted from the prescription input device 23. Next, a medicine is supplied in the medicine supplying section 14.

The medicine supplying section 14 holds and stores a predetermined kind of medicines in the form of a glass bottle, a bottle, an ampule, a vial, and the like in a predetermined supply medicine storage device 31. In this medicine supplying section 14, a medicine is supplied from the supply medicine storage device 31 in accordance with the prescription direction of the prescription generating section 21. The supplied medicine is then inserted into the medicine container 92. The medicine supplying section 14 records supply information, related to a name of the supply medicine and the number thereof, into the management information of the dispensation managing section 11. Further, along with the supply information, the medicine supplying section 14 records the prescription code of the prescription direction and an ID code of the medicine container 92 into the management information of the dispensation managing section 11. It is to be noted that the prescription direction to the medicine supplying section 14 can also be communicated from the prescription generating section 21 to the medicine supplying section 14 through the dispensation managing section 11.

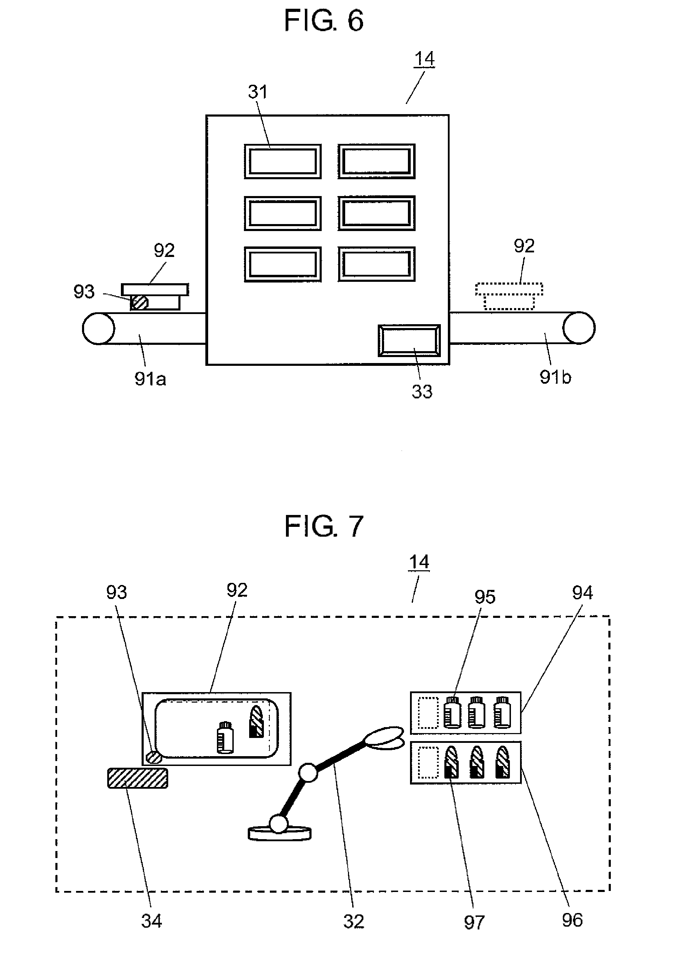

FIG. 6 is a schematic constitutional view of the medicine supplying section 14, and FIG. 7 is a view for explaining a medicine supply of the medicine supplying section 14.

As shown in FIG. 6, the medicine supplying section 14 has six supply medicine storage devices 31. A plurality of units of medicines of the same kind are stored in each supply medicine storage device 31. A belt conveyer 91a is configured so as to bring the medicine container 92 placed thereon into the medicine supplying section 14.

Herein, an RFID tag 93 attached to the medicine container 92 records an ID code provided with respect to each medicine container 92. After the supply has been completion herein, the supply communication device 33 of the medicine supplying section 14 communicates, to the management communication device 25 of the dispensation managing section 11, supply information related to a name of a supply medicine, the number thereof and a supply time, along with the prescription code of the prescription direction and the ID code of the medicine container 92.

The management information of the dispensation managing section 11 is then updated. The belt conveyer 91b carries the medicine container 92, having been supplied with the medicines in the medicine supplying section 14, to the outside of the medicine supplying section 14.

This supply operation of the medicine supplying section 14 is described. As shown in FIG. 7, the robot arm 32 moves in accordance with a prescription direction. For example, one piece of medicine 95 stored in the supply medicine storage device 94 is taken out and inserted into the medicine container 92, and one piece of medicine 97 stored in the supply medicine storage device 96 is taken out and inserted into the medicine container 92.

The RFID reader 34 reads the ID code of the RFID tag 93 of the medicine container 92, and transfers the ID code to the supply communication device 33. Herein, the RFID reader 34 for reading the RFID tag 93 of the medicine container 92 is an ID code reader for reading the ID code of the medicine container 92. In addition, the RFID reader 34 is also a reader of the identification tag element.

To the RFID tag 93, not only an RFID tag recorded with an ID code is applied, but a one-dimensional bar code, a two-dimensional bar code, or the like can also be applied. Changing the RFID reader 34 to a bar code reader for reading a bar code is also included in the present invention.

Further, to a mechanism for taking the medicines 95, 97 out of the supply medicine storage devices 94, 96, not only the articulated type robot arm 32 is applied, but another mechanism for taking out a medicine, such as an orthogonal type robot, may be appropriately applied. Each device in this medicine supplying section 14 is controlled by the supply processing device 35 which is connected to each component.

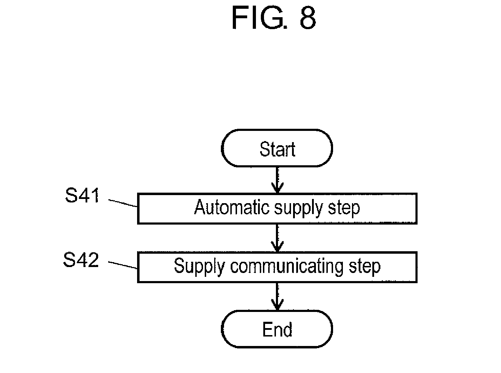

FIG. 8 is a flowchart for medicine supply performed in the medicine supplying section 14. As shown in FIG. 8, in the medicine supplying section 14, an automatic supply step S41 is performed for taking supply medicines out of the supply medicine storage devices 94, 96 in accordance with the prescription direction, and inserting the medicines into the medicine container 92. Then a supply communicating step S42 is performed for making the ID code of the medicine container 92 and the prescription code of the prescription direction have a corresponding relation to each other, to communicate the corresponding relation and supply information regarding the medicines inserted into the medicine container 92 to the dispensation managing section 11.

After the medicine supply has been performed in the medicine supplying section 14, medicines are procured in the medicine procuring section 15. The medicine procuring section 15 is an apparatus for comparing the medicine container 92 taken out of the medicine supplying section 14 with the dispensation direction information, to replenish a medicine lacking in the medicine container 92. Medicines in the dispensation direction information may include medicines corresponding to powerful medicines or poisons, and these are management medicines that require the strict management. It is to be noted that the powerful medicine means a medicine having a vigorous pharmacological action.

These management medicines cannot be automatically supplied with ease from the medicine supplying section 14. Therefore, these medicines are strictly managed in the medicine procuring section 15. These medicines are required to be replenished and procured by a limited dispensation operator such as a pharmacist or a nurse. Further, an infusion bag with its temperature needed to be controlled is also required to be replenished and procured in the medicine procuring section 15. It is to be noted that each device of the medicine procuring section 15 is controlled by the procurement processing device 46.

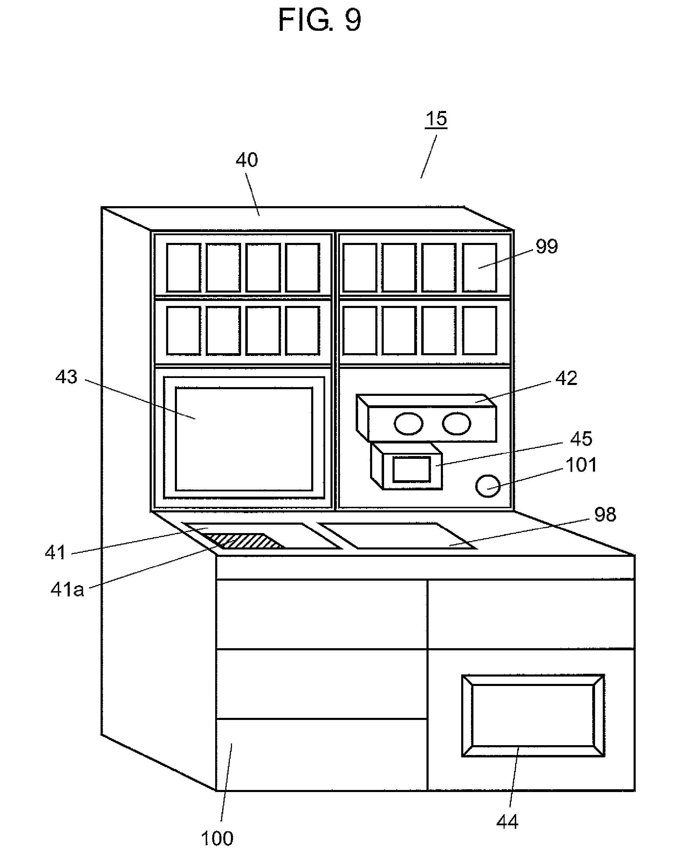

FIG. 9 is a schematic constitutional view of the medicine procuring section 15. The medicine container 92 is carried by a dispensation operator to the medicine procuring section 15. The dispensation operator is subjected to a procurement person authentication by means of an iris recognition with use of the person authentication device 42.

First, the person authentication device 42 compares operator information on the dispensation operator, obtained by the iris recognition, with register information. It is then checked whether or not the dispensation operator is a dispensation operator permitted for a replenishment and a procurement, namely a procurement person. The register information are iris information, positions, names, and the like of the pharmacists and the nurses permitted for the operation. When a non-registered dispensation operator performs the authenticating operation, letters "not-permitted" are displayed on the procurement display 43. When a registered dispensation operator performs the authenticating operation, letters "permitted" are displayed on the procurement display 43.

Herein, to the authentication method performed with the person authentication device 42, not only the iris recognition is applied, but a fingerprint recognition, a vein recognition or the like can also be applied. Further, a person authentication by means of a security card is also possible although the authentication operation therewith necessitates a little time and effort, and is included in the present invention. It is to be noted that the person recognition is performed by means of the iris recognition in the present embodiment with the aim of constantly and reliably performing the person authentication in consideration of a case where both hands of a procurement person are occupied, a case where the hands are dirty, and some other case.

The procurement person permitted for the operation places the medicine container 92 on the dedicated medicine container placement section 41. The RFID reader 41a reads the RFID tag 93 of the medicine container 92, to obtain the ID code of the medicine container 92. The procurement communication device 44 communicates with the management communication device 25 of the dispensation managing section 11, to obtain the management information corresponding to the ID code of the medicine container 92.

It should be noted that the medicine procuring section 15 has a container correspondence display 98 for visually supporting procurement of a medicine. Further, the medicine procuring section 15 has a management medicine shelf case 99 for holding management medicines therein, and a cool shelf case 100 for holding cool medicines that needs to be kept cool therein. Moreover, the medicine procuring section 15 has a button 101 for inputting that the dispensation operator has completed the medicine procurement, or has completed the procurement inspection.

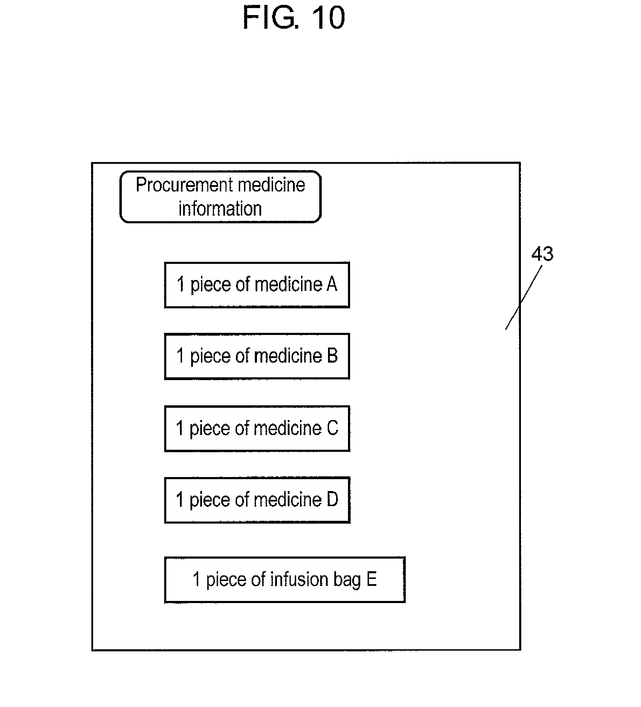

FIG. 10 is a view showing a display screen of the procurement display 43 of the medicine procuring section 15. Based upon the management information of the dispensation managing section 11, the supplementary medicine information on the medicine container 92 placed on the medicine container placement section 41 is displayed on the procurement display 43. FIG. 10 shows the display screen of the procurement display 43. In the case as shown in FIG. 10, medicines to be procured are one piece of medicine A, one piece of medicine B, one piece of medicine C, one piece of medicine D, and one piece of infusion bag E.

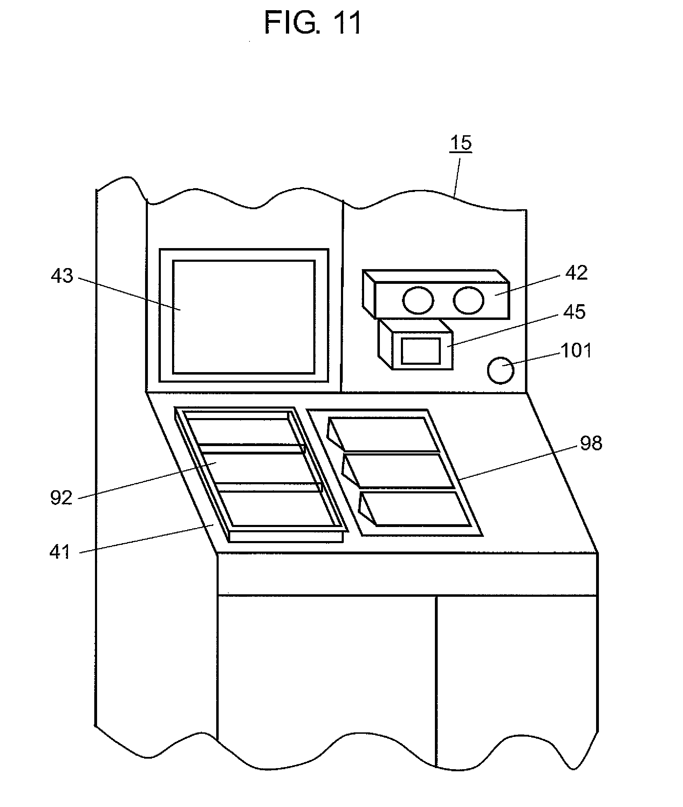

FIG. 11 is a view showing a brief overview of the container correspondence display 98. As shown in FIG. 11, in the container correspondence display 98, where and which medicines are to be procured are displayed with display contents corresponding to a size and division of the medicine container 92. Therefore, the container correspondence display 98 displays in a tripartite manner as shown in FIG. 11,and each of triangle marks points each of divided places of the medicine container 92.

A procuring operation in the medicine procuring section 15 is described. First, when a procurement person is permitted by the person authentication device 42, the locks of the management medicine shelf case 99 and the cool shelf case 100 of medicine procurement shelf 40 are released. The procurement person takes required medicines out of the management medicine shelf case 99 and the cool shelf case 100 of the medicine procurement shelf 40, while referring to the display of the procurement display 43 and the container correspondence display 98. The procurement person checks a glass bottle or the like of each of the medicines having been taken out to see whether or not each of the medicines is a correct supplementary procurement medicine through the bar code reader 45. When the medicine, read with the bar code reader 45, corresponds to the supplementary procurement medicine, the procurement display 43 and the container correspondence display 98 display letters "correct", and when the medicine does not correspond to the supplementary procurement medicine, those displays display letters "wrong". When the procurement display 43 and the container correspondence display 98 display letters "correct", the procurement person inserts the medicine, having been taken out, into a corresponding place to the medicine container 92.

Herein, as for the medicine read with the bar code reader 45, changing a color of a name of the corresponding medicine in the container correspondence display 98 can facilitate to inform the procurement person of which medicine is currently treated. Further, changing a color of a frame of divided display contents corresponding to the divided place of the medicine container 92 can also facilitate to inform the procurement person of which divided place the medicine is to be procured in.

Upon the completion of the procurement of all the medicines, the procurement person presses the button 101, to complete the procuring operation. The procurement communication device 44 communicates with the management communication device 25 of the dispensation managing section 11. The procurement information including the procurement person information is communicated to the management communication device 25 of the dispensation managing section 11, along with the ID code of the medicine container 92. The management information is then updated.

Herein, at least some of a name of the supplementary procurement medicine, the number thereof, and a procurement time and date thereof may be included in the procurement information as necessary. Further, the bar code reader 45 is a medicine code reader for reading a code of a medicine. To the form of a medicine code of a medicine, not only the form of the bar code stuck to a glass bottle or the like is applied, but also a form of the medicine code recorded into another recording body, such as RFID, may be applied, and these configurations are also included in the present invention.

Next, the operation is shifted to a procurement inspecting operation. A dispensation operator is subjected to a procurement inspector authentication by means of an iris recognition with use of the person authentication device 42. The person authentication device 42 compares dispensation operator information of the dispensation operator with register information, to check whether or not the dispensation operator is a dispensation operator permitted for a procurement inspection, namely a procurement inspector.

When a non-registered dispensation operator performs the authenticating operation, letters "not-permitted" are displayed on the procurement display 43. When a registered dispensation operator performs the authenticating operation, letters "permitted" are displayed on the procurement display 43.

When the procurement inspector receives permission for the operation, the procurement communication device 44 obtains from the dispensation managing section 11 management information corresponding to the ID code of the medicine container 92 obtained with use of the RFID reader 34. Based upon the management information of the dispensation managing section 11, procurement medicine information of the medicine container 92 placed on the medicine container placement section 41 is displayed on the procurement display 43 and the container correspondence display 98. This display system is similar to displays for the procurement shown in FIGS. 10 and 11. However, the display contents are different from those in FIGS. 10 and 11 in that a pre-procured medicine previously procured in the medicine container 92 is displayed.

The procurement inspector compares the display in the procurement display 43 or the container correspondence display 98 with the procurement medicines in the medicine container 92, to make a check. When the medicine procurement has been correctly performed, the procurement inspector presses the button 101, to complete the procurement inspection. As this button 101 used can be a touch panel section installed in part of the container correspondence display 98, a dedicated input button, or the like. The procurement communication device 44 communicates procurement inspection information including procurement inspector information to the management communication device 25 of the dispensation managing section 11, along with the ID code of the medicine container 92. The dispensation managing section 11 then updates the management information.

Herein, a name of the procurement medicine, the number thereof, a procurement inspection time and date thereof, or the like may be included in the procurement inspection information as necessary.

It is to be noted that, although the procurement inspector is not required to be subjected to the person authentication in the case of the procurement person and the procurement inspector being an identical person, it is preferable to perform another person authenticating operation in the sense of dividing the operation.

Further, in order to reduce procurement errors, it is preferable that the procurement person and the procurement inspector be different persons.

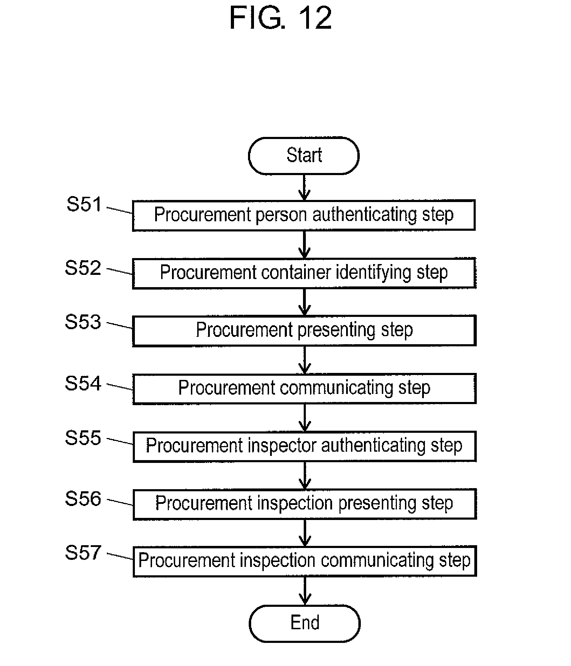

FIG. 12 is a flowchart for a medicine procurement and a procurement inspection in the medicine procuring section 15. As shown in FIG. 12, in the medicine procuring section 15, a procurement person authenticating step S51 is performed for comparing the dispensation operator information of the dispensation operator with the register information, to make a check. The procurement container identifying step S52 is then performed for identifying the ID code of the medicine container 92.

Subsequently, based upon the management information of the dispensation managing section 11 corresponding to the ID code of the medicine container 92, a procurement presenting step S53 is performed for showing the supplementary medicine information on the medicine container 92. A procurement communicating step S54 is performed for communicating the procurement information, including the procurement person information, to the dispensation managing section 11, while making the procurement information correspond to the ID code of the medicine container 92.

Subsequently to the medicine procurement in the medicine procuring section 15, a procurement inspection is performed. In the medicine procuring section 15, a procurement inspector authenticating step S55 is performed for authenticating a procurement inspector and making a check by comparison with register information. Based upon the management information of the dispensation managing section 11 corresponding to the ID code of the medicine container 92, a procurement inspection presenting step S56 is performed for showing information of the procurement medicines procured in the medicine container 92. A procurement inspection communicating step S57 is performed for communicating the procurement inspection information, including the procurement inspector information, to the dispensation managing section 11, while making the procurement inspection information correspond to the ID code of the medicine container 92.

Herein, either the procurement person authenticating step S51 or the procurement container identifying step S52 may be performed prior to the other. Further, the procurement communicating step S54 may be performed simultaneously with the procurement inspection communicating step S57. In the case of the procurement person and the procurement inspector being identical, the procurement inspector authenticating step S55 can be omitted.

After the medicine procurement and the procurement inspection have been performed in the medicine procuring section 15, a medicine mixture and a mixture inspection are performed in the medicine mixing section 16. The medicine mixing section 16 is configured for mixing medicines procured in the medicine container 92 as necessary. The dispensation direction information of the prescription direction includes names of medicines to be mixed and a quantity thereof. The direction information is, for example, the direction of mixing a required quantity of a poisonous medicine into an infusion bag, or the like. Each device in this medicine mixing section 16 is controlled by the mixture processing device 56.

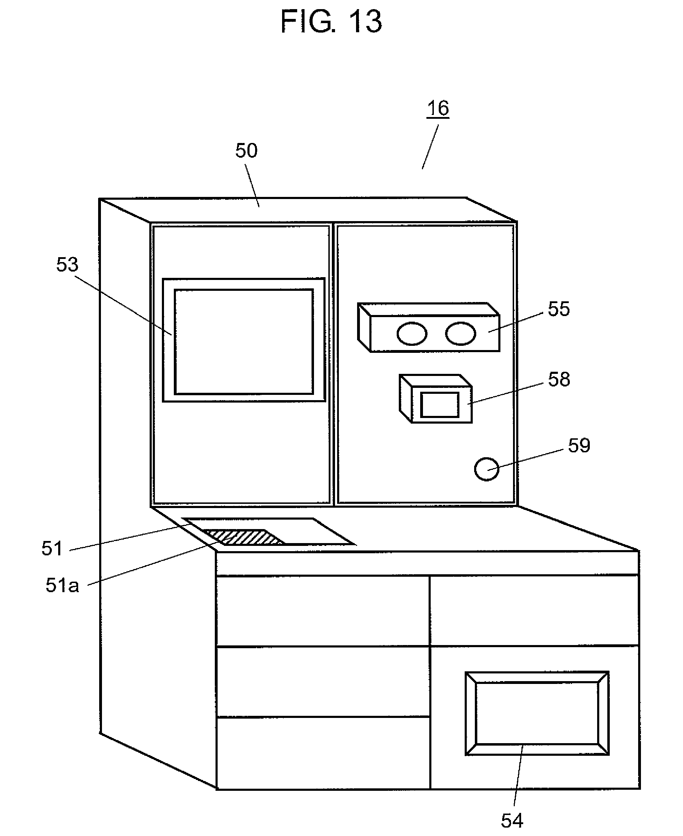

FIG. 13 is a schematic constitutional view of the medicine mixing section 16. The medicine container 92 is carried by a dispensation operator to the medicine mixture shelf 50 of the medicine mixing section 16. The dispensation operator is subjected to a mixing person authentication by means of an iris recognition with use of the person authentication device 52. The person authentication device 52 compares the operator information of the dispensation operator with register information, to check whether or not the dispensation operator is a dispensation operator permitted for mixing operation, namely a mixing person. In the register information registered are iris information, positions, names, and the like of the pharmacists and the nurses permitted for the operation each as the mixing persons. When a non-registered dispensation operator performs the authenticating operation, letters "not-permitted" are displayed on the mixture display 53. When a registered dispensation operator performs the authenticating operation, letters "permitted" are displayed on the mixture display 53.

The mixing person permitted for operation places the medicine container 92 on the medicine container placement section 51 as the dedicated place. The RFID reader 51a reads the RFID tag 93 of the medicine container 92, to obtain the ID code of the medicine container 92. The mixture communication device 54 communicates with the dispensation managing section 11, to obtain the management information corresponding to the ID code of the medicine container 92. Based upon the management information of the dispensation managing section 11, the mixture medicine information on the medicine container 92 placed on the medicine container placement section 51 is displayed on the mixture display 53.

Herein, as a display of the mixture medicine information, names of medicines to be mixed, the number thereof, and a mixture procedure therefore are displayed. In addition, a quantity of a medicine may be indicated in place of the number thereof.

The mixing person performs the operation in accordance with the mixture medicine information presented in the mixture display 53. The mixing person takes a medicine out of the medicine container 92, and checks the name of the medicine with the bar code reader 55. The mixture medicine information is then presented in the mixture display 53, and the mixing person mixes the medicine in accordance with the presented mixture information. For example, the mixing person draws a designated quantity of injection into an injector (not shown). Next, the mixing person takes an infusion bag (not shown) out of the medicine container 92, reads the bar code of the infusion bag with the bar code reader 55, to check the name of the medicine. The medicine inside the injector is then injected into this infusion bag. Accordingly, the infusion bag is mixed with the medicine. The mixing person returns the infusion bag mixed with the medicine to the medicine container 92, and disposes of the remaining medicine.

Upon completion of the medicine mixture, the mixing person presses the button 59, to complete the mixing operation. As this button 59 used can be a touch panel section installed in part of the mixture display 53, a dedicated input button, or the like. The mixture communication device 54 communicates with the dispensation managing section 11. The mixture information including the mixing person information and the mixing operation contents is communicated to the dispensation managing section 11, along with the ID code of the medicine container 92. The dispensation managing section 11 then updates the management information. Herein, in the mixture information including the mixing information, the names of the mixture medicine, the number thereof, the quantity thereof, the mixture time and date thereof and the like may be included as necessary.

Next, the operation is shifted to a mixture inspecting operation. A dispensation operator is subjected to a mixing inspector authentication by means of an iris recognition with use of the person authentication device 52 for performing the iris recognition. The person authentication device 52 compares operator information of the dispensation operator with register information, to check whether or not the dispensation operator is a dispensation operator permitted for the mixture inspection, namely a mixing inspector. When a non-registered dispensation operator performs the authenticating operation, letters "not-permitted" are displayed on the mixture display 53. When a registered dispensation operator performs the authenticating operation, letters "permitted" are displayed on the mixture display 53.

When the mixing inspector receives a permission for the operation, the mixture communication device 54 communicates the ID code of the medicine container 92, obtained with use of the RFID reader 51a, to the dispensation managing section 11. The management information corresponding to the ID code of the medicine container 92 is then obtained. Based upon the management information of the dispensation managing section 11, the mixture inspection medicine information of the medicine container 92 placed on the medicine container placement section 51 is displayed on the mixture display 53.

The mixing inspector checks the mixture medicine in the medicine container 92 with reference to the mixture inspection medicine information in the mixture display 53. When the mixture inspection medicine information displayed on the mixture display 53 agrees with the mixture medicine in the medicine container 92, the mixing inspector presses the button 59, to complete the mixture inspection operation. The mixture communication device 54 communicates the mixture inspection information including the mixing inspector information to the management communication device 25 of the dispensation managing section 11, along with the ID code of the medicine container 92. The dispensation managing section 11 then updates the management information.

Herein, the name of the mixture medicine, the checked place thereof, the mixture inspection time and date thereof, or the like may be included in the mixture inspection information as necessary. It is to be noted that the mixing inspector is not required to be subjected to the person authentication in the case of the mixing person and the mixing inspector being an identical person. However, it is preferable to perform another person authentication in the sense of dividing the operation. Further, in order to reduce mixture errors, it is preferable that the mixing person and the mixing inspector be different persons. While the mixture inspection medicine information may be presented as necessary, it is preferable to display check items and check points as the mixture inspection medicine information. This leads to prevent leakages of the mixture inspection.

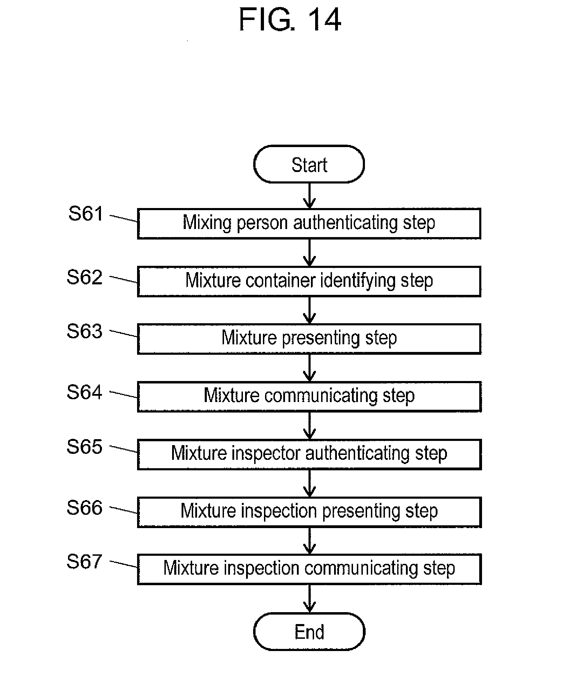

FIG. 14 is a flowchart for the medicine mixture and the mixture inspection in the medicine mixing section 16. As shown in FIG. 14, in the medicine mixing section 16, a mixing person authenticating step S61 is performed for authenticating a mixing person and making a check by comparison with register information. Next, a mixture container identifying step S62 is performed for obtaining the ID code of the medicine container 92. Subsequently, based upon the management information of the dispensation managing section 11 corresponding to the ID code of the medicine container 92, a mixture presenting step S63 is performed for showing the mixture medicine information of the medicine container 92. Thereafter, a mixture communicating step S64 is performed for communicating the mixture information, including the mixing person information, to the dispensation managing section 11, while making the mixture information correspond to the ID code of the medicine container 92.

Subsequently to the mixing operation in the medicine mixing section 16, the mixture inspection operation is performed. In the medicine mixing section 16, a mixing inspector authenticating step S65 is performed for authenticating a mixing inspector and making a check by comparison with register information. Next, based upon the management information of the dispensation managing section 11 corresponding to the ID code of the medicine container 92, a mixture inspection presenting step S66 is performed for showing the mixture inspection medicine information of the mixture medicine on the medicine container 92. A mixture inspection communicating step S67 is performed for communicating the mixture inspection information, including the mixing inspector information, to the dispensation managing section 11, while making the mixture inspection information correspond to the ID code of the medicine container 92.

Herein, either the mixing person authenticating step S61 or the mixture container identifying step S62 may be performed prior to the other. Further, the mixture communicating step S64 may be performed simultaneously with the mixture inspection communicating step S67. In the case of the mixing person and the mixing inspector being identical, the mixing inspector authenticating step S65 can be omitted.

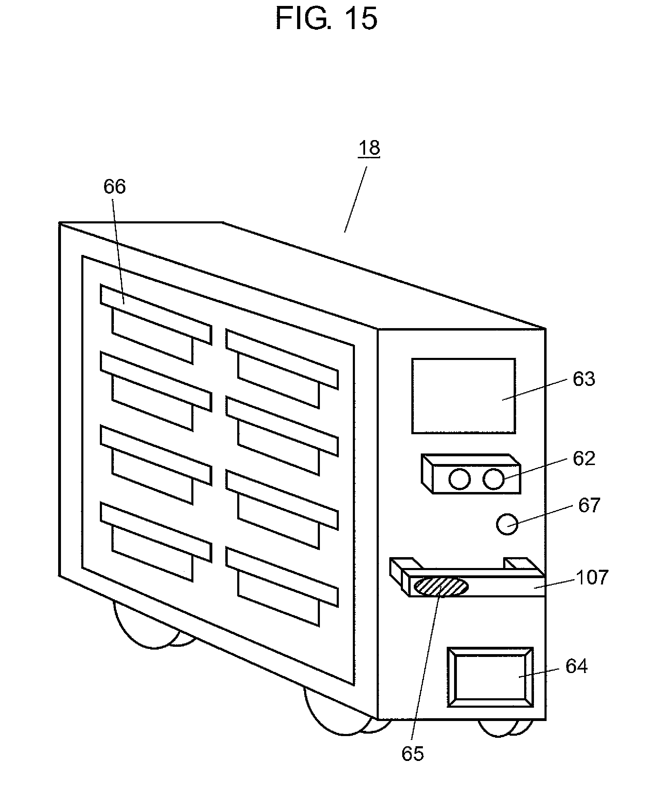

FIG. 15 is a schematic constitutional view of the medicine delivering section 18. As shown in FIG. 15, the medicine delivering section 18 is an apparatus for placing the medicine container 92 thereon, to deliver the medicine container 92 to a hospital ward, or one among the apparatuses of the medicine management system 12.

The medicine container 92 carried to the hospital ward is not necessarily one on which the medicine mixture and the mixture inspection have been completed in the medicine mixing section 16. The medicine container 92 not required for the mixing operation is moved from the medicine procuring section 15 directly to the medicine delivering section 18. The medicine container 92 not required for the replenishing and procuring operations and the mixing operation is moved from the medicine supplying section 14 directly to the medicine delivering section 18. A plurality of these medicine containers 92 are mixed and moved to the medicine delivering section 18, and carried to the hospital ward and the like.

In the embodiment 1, a delivery cart is used as the medicine delivering section 18. A dispensation operator (not shown) makes the medicine container 92 close to the RFID reader 65 provided on a handle 107 of the medicine delivering section 18. The RFID reader 65 reads the RFID tag 93 of the medicine container 92, to obtain the ID code of the medicine container 92. The medicine delivering section 18 communicates with the dispensation managing section 11 through the delivery communication device 64, to obtain the management information corresponding to the ID code of the medicine container 92.

Based upon the management information of the dispensation managing section 11, a delivery permission/non-permission information on the medicine container 92 is displayed on the delivery display 63.

Herein, the delivery permission/non-permission information is information indicating whether delivery of the medicine container 92 is permitted or not permitted. When the delivery is not permitted, which operation has not been conducted is displayed on the delivery display 63. The medicine container 92 not permitted for the delivery is returned to a dedicated holding place (not shown) for the non-conducted operation. In addition, each device of the medicine delivering section 18 is controlled by the delivery processing device 68.

The dispensation operator stores the medicine container 92 permitted for the delivery into the delivery shelf 66 of the medicine delivering section 18. The dispensation operator is authenticated as a delivery person by means of an iris recognition with use of the person authentication device 62. The person authentication device 62 compares operator information of the dispensation operator with register information, to check whether or not the dispensation operator is a dispensation operator permitted for the delivery operation. The register information may be iris information, positions, names, and the like of the delivery persons permitted for the operation. The medicine delivering section 18 is under breaking and in a stopped state.

When a non-registered dispensation operator performs the authenticating operation, the medicine delivering section 18 displays letters "not-permitted" in the delivery display 63. Further, a brake is locked so that the medicine delivering section 18 is kept in the stopped state. When a registered dispensation operator performs the authenticating operation, the medicine delivering section 18 displays letters "permitted" in the delivery display 63, and the medicine delivering section 18 is made movable by the delivery person.

When a predetermined number of the medicine containers 92 are placed, the delivery person delivers the medicine delivering section 18 to the hospital ward. Further, once the medicine container 92 not permitted for the delivery is detected, the delivery operation is permitted by the person authentication device 62. Subsequently, unless the delivery person presses a reset button 67, the medicine delivering section 18 is kept in the stopped state with the brake being locked. The delivery communication device 64 communicates the delivery person information and the delivery information, including a delivery time, to the management communication device 25 of the dispensation managing section 11. The dispensation managing section 11 then updates the management information.

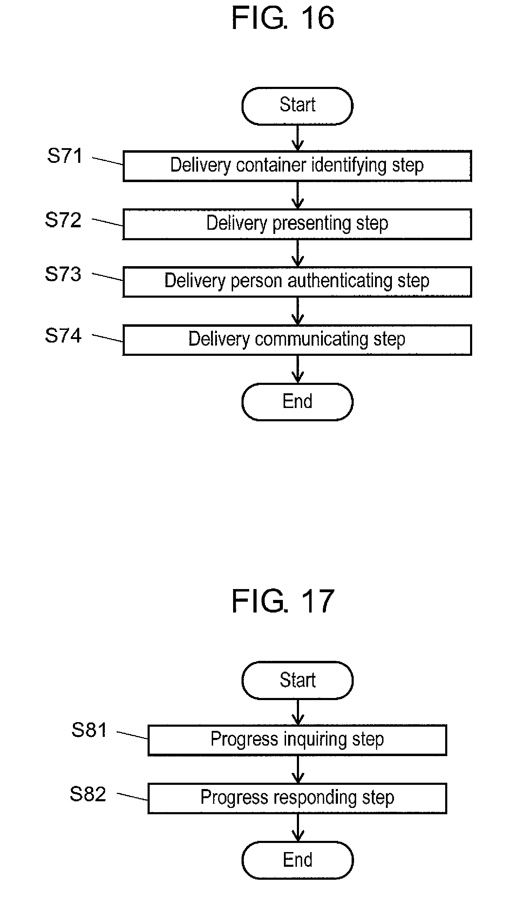

FIG. 16 is a flowchart for the delivery operation performed in the medicine delivering section 18. As shown in FIG. 16, in the medicine delivering section 18, a delivery container identifying step S71 is performed for obtaining the ID code of the medicine container 92. Next, based upon the management information of the dispensation managing section 11 corresponding to the ID code of the medicine container 92, a delivery presenting step S72 is performed for showing the delivery permission/non-permission information of the medicine container 92. Subsequently, a delivery person authenticating step S73 is performed for comparing the operator information of the dispensation operator with the register information, to make a check. Thereafter, a delivery communicating step S74 is performed for communicating delivery information, including delivery person information and cart information, to the dispensation managing section 11, while making the delivery information correspond to the ID code of the medicine container 92.

Herein, the delivery person authenticating step S73 may be performed before the delivery container identifying step S71. Further, the delivery container identifying step S71 and the delivery presenting step S72 may be repeatedly performed until a predetermined number of the medicine containers 92 are placed on the medicine delivering section 18, and thereafter, the delivery person authenticating step S73 and the delivery communicating step S74 may be performed.

It is to be noted that the medicine supplying section 14, the medicine procuring section 15, the medicine mixing section 16, and the medicine delivering section 18 described above are the dispensation operating sections of the medicine management system 12. Further, the medicine supply, medicine procurement, the procurement inspection, the medicine mixture, the mixture inspection and the medicine delivery are the dispensation operations performed in the dispensation operating section. Moreover, the automatic supply in the medicine supplying section 14 and the medicine procurement in the medicine procuring section 15 are to collect medicines into the container, and may thus be considered as the medicine collecting operations.

Although the medicine supplying section 14 described above is described with regard to the automatic supply of an injection, an automatic powder packaging machine and an automatic tablet packaging machine are also considered as the medicine supplying section 14. Hence there may be cases where two medicine supplying sections 14 are present.

The progress inquiring section 22 shown in FIG. 4 is an apparatus for an inquiry person such as a doctor, a nurse or a pharmacist to inquire about the progress status of the dispensation operation. The progress inquiring section 22 includes the inquiry input device 71 and the inquiry display 72. An inquiry person inputs dispensation inquiry information including the prescription code of the prescription direction with use of the inquiry input device 71, to communicate the information to the dispensation managing section 11 through the inquiry communication device 73. Based upon the management information on the medicine container 92 made to correspond to the prescription code, the dispensation managing section 11 communicates the dispensation progress information corresponding to the prescription direction to the progress inquiring section 22. The inquiry display 72 of the progress inquiring section 22 displays the dispensation progress information of the dispensation managing section 11. Herein, the dispensation progress information is the information representing the progress status of the dispensation based upon the management information of the dispensation managing section 11 which corresponds to the ID code of the medicine container 92. The information are, for example, the completion of the medicine supply, the completion of the medicine procurement, the completion of the medicine mixture, in process of the delivery, and the like.

FIG. 17 is a flowchart for the progress inquiry made in the progress inquiring section 22. The in progress inquiring section 22, a progress inquiring step S81 is performed for communicating the dispensation inquiry information, including the prescription code of the prescription direction, to the dispensation managing section 11. Based upon the management information of the dispensation managing section 11, a progress responding step S82 is performed for informing the dispensation progress information corresponding to the prescription direction.

The dispensation managing section 11 shown in FIG. 4 is an apparatus for recording and managing a variety of information on a series of dispensation operations. The dispensation managing section 11 communicates with each of the prescription generating section 21, the medicine supplying section 14, the medicine procuring section 15, the medicine mixing section 16, the medicine delivering section 18, the medicine withdrawing section 17 and the progress inquiring section 22, to update the required information. The management communication device 25 is linked through a communication network, to the prescription communication device 24, the supply communication device 33, the procurement communication device 44, the mixture communication device 54, the delivery communication device 64, the withdrawal communication device 84 and the inquiry communication device 73. In addition, the communication network may be wired or wireless.

The management processing device 27 processes information obtained from devices of the medicine management system 12, and makes the information correspond to the ID code of the medicine container 92, to update the prescription code and the management information in the management storage device 26. The management information includes the dispensation direction information, the supply information, the procurement information, the procurement inspection information, the mixture information, the mixture inspection information, the delivery information, and the withdrawal information.

The management processing device 27 passes information of the management storage device 26 to the management communication device 25, and communicates a variety of information with the prescription generating section 21, the medicine supplying section 14, the medicine procuring section 15, the medicine mixing section 16, the medicine delivering section 18, the medicine withdrawing section 17 and the progress inquiring section 22.

Thereby, the prescription code, the dispensation direction information, the supply information, the procurement information, the procurement inspection information, the mixture information, the mixture inspection information, the withdrawal information, the delivery information and the inquiry information, which are made to correspond to the ID code of the medicine container 92, are updated as part of the management information in management storage device 26. It is to be noted that in the embodiment 1, storage medicine information is created from the dispensation direction information, the supply information, the procurement information, the procurement inspection information, the mixture information, the mixture inspection information, the withdrawal information and the delivery information, and recorded into the management information. It should be noted that the storage medicine information is medicine names of medicines, held in the medicine container, and the number thereof.

In addition, the register information on the person authentication devices in the medicine procuring section 15, the medicine mixing section 16, the medicine delivering section 18 and the medicine supplying section 14 may be stored in the dispensation managing section 11, and the comparison with the authentication information in the person authentication devices may be performed in the management processing device 27.

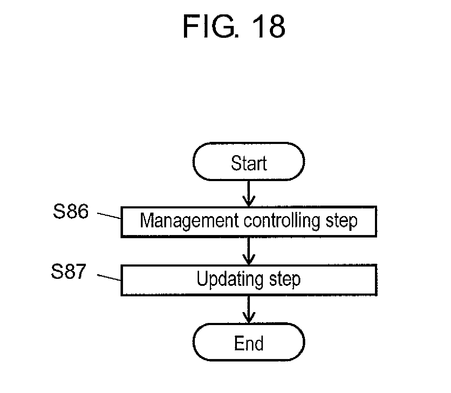

FIG. 18 is a flowchart for information update of the dispensation managing section 11. In the dispensation managing section 11, a management controlling step S86 is performed for communicating with the medicine supplying section 14, the medicine procuring section 15, the medicine mixing section 16, the medicine delivering section 18, the progress inquiring section 22 and the medicine withdrawing section 17. An updating step S87 is performed for updating, as the management information, the prescription code, the dispensation direction information, the supply information, the procurement information, the procurement inspection information, the mixture information, the mixture inspection information, delivery information, the withdrawal information and the like, while making those information correspond to the ID code of the medicine container 92.

As thus described, according to the medicine management method using the medicine management system 12, it is possible to manage the dispensation information regarding the dispensation operation and manage the progress of each dispensation operation with respect to each medicine container 92. Thereby, even when the dispensation operation has been divided into a plurality of operations, it is possible to accurately grasp contents of the dispensation operation performed in each dispensation operating section.

The medicine withdrawing section 17 shown in FIG. 4 is an apparatus for withdrawing a cancelled medicine in the case of a cancellation of the whole or part of the prescription direction. The medicine withdrawing section 17 has the withdrawal processing device 85 and the medicine withdrawal shelf 80. The withdrawal processing device 85 receives a cancellation of a prescription made by a doctor through the withdrawal communication device 84. Based upon the prescription code, that the whole or part of the prescription contents of the prescription direction has been cancelled is communicated to the management communication device 25 of the dispensation managing section 11. Thereby, the dispensation managing section 11 obtains the cancelled prescription code and cancellation information. Herein, as the cancellation information, there is an entire cancellation or a partial cancellation.

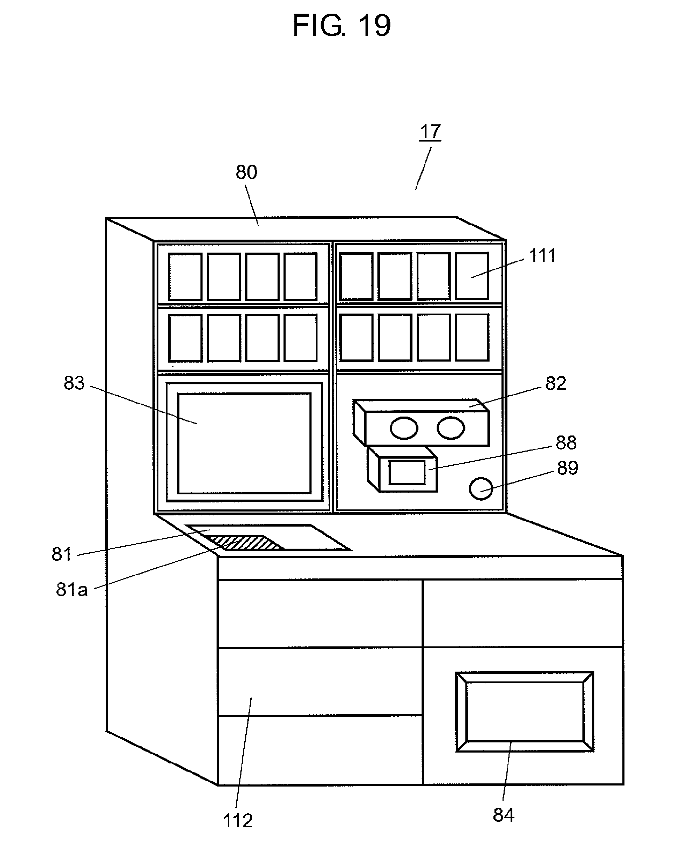

FIG. 19 is a schematic constitutional view of the medicine withdrawing section 17. The medicine container 92 is carried by the dispensation operator to the medicine withdrawal shelf 80. The person authentication device 82 authenticates a withdrawal person by means of an iris recognition. The person authentication device 82 compares authentication information of the dispensation operator with the register information, to check whether or not the dispensation operator is a dispensation operator permitted for a medicine withdrawal, namely a withdrawal person. The register information includes the iris information, the positions, the names, and the like of the pharmacists and the nurses permitted for the operation. When a non-registered dispensation operator performs the authenticating operation, letters "not-permitted" are displayed on the withdrawal display 83. When a registered dispensation operator performs the authenticating operation, letters "permitted" are displayed on the withdrawal display 83, and the following operation is permitted. Each device of the medicine withdrawing section 17 is controlled by the withdrawal processing device 85.

The withdrawal person sets the medicine container 92 on the medicine container placement section 81 as the dedicated place. The RFID reader 81a reads the RFID tag 93 of the medicine container 92, to obtain the ID code of the medicine container 92. The withdrawal communication device 84 communicates with the management communication device 25, to obtain the management information corresponding to the ID code of the medicine container 92 from the dispensation managing section 11. Then, based upon the management information of the dispensation managing section 11, the medicine withdrawal information on the medicine container 92 placed is displayed on the withdrawal display 83. The medicine withdrawal information is a variety of information for withdrawing a medicine.

At this time, on the withdrawal display 83, the cancellation information including a name of a cancelled medicine and the number thereof is presented as the medicine withdrawal information.

When the operation of the withdrawal person is permitted by the person authentication device 82, the locks of a management medicine withdrawal case 111 and a requiring cool medicine withdrawal case 112 in the medicine dispensation shelf 80 are released. The withdrawal person takes a medicine to be withdrawn out of the medicine container 92, while referring to the display of the withdrawal display 83, and makes the bar code reader 88 read the bar code of the medicine which is a glass bottle or the like.

When the medicine, read with the bar code reader 88, corresponds to a withdrawal medicine, the withdrawal display 83 displays letters "withdrawal medicine" as the medicine withdrawal information. When the medicine does not correspond to a withdrawal medicine, the withdrawal display 83 displays letters "disposal medicine" or "non-cancelled medicine" as the medicine withdrawal information. The withdrawal person puts the withdrawal medicine, taken out in the case of the letters being "withdrawal medicine", into the required shelf case of the management medicine withdrawal case 111 or the requiring cool medicine withdrawal case 112.

In the case of the letters being "disposal medicine", the withdrawal person performs a disposal process on the medicine in accordance with a disposal direction of the medicine withdrawal information. Upon the completion of the withdrawal operation on the medicine, the withdrawal person presses a button 89, to complete the withdrawal operation. The withdrawal communication device 84 communicates withdrawal operation information, including withdrawal person information to the dispensation managing section 11, along with the ID code of the medicine container, to update the management information of the management storage device 26. Herein, the name of the withdrawal medicine, the number thereof, the withdrawal time and date thereof, or the like may be included in the withdrawal operation information.

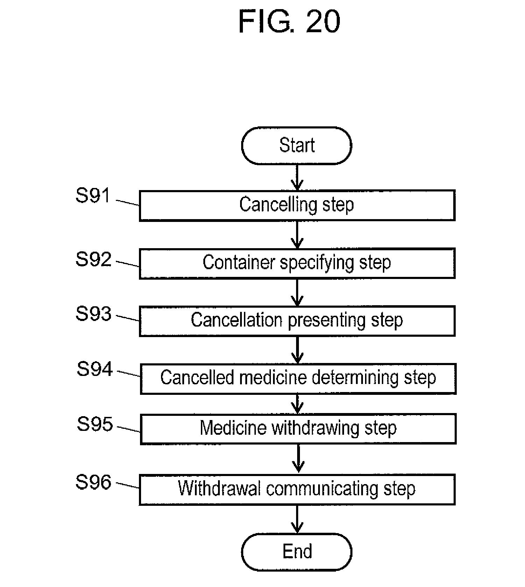

FIG. 20 is a flowchart showing a brief overview of the medicine withdrawing step S03. First, a cancelling step S91 is performed for communicating to the dispensation managing section 11 that the whole or part of the prescription contents of the prescription direction has been canceled. Next, a container specifying step S92 is performed for obtaining the ID code of the medicine container 92 which corresponds to the prescription code, based upon the management information of the dispensation managing section 11 which includes the prescription code.

When a cancelled medicine container 92 is identified in the medicine dispensation shelf 80, a cancellation presenting step S93 is performed for showing cancellation information on the medicine container 92.

The withdrawal person detects the medicine information on the cancelled medicine by means of the bar code with reference to this cancellation display. Then, a determination result as to whether or not each detected medicine is a withdrawal medicine is showed as medicine withdrawal information in the withdrawal display 83.

Thereafter, a cancelled medicine determining step S94 is performed for determining whether or not the cancelled medicine in the medicine container 92 corresponds to a withdrawal medicine, based upon the management information on the medicine container 92. Based upon the result of the determination, the medicine withdrawal information is created. A medicine withdrawing step S95 is then performed for withdrawing the cancelled medicine in accordance with the medicine withdrawal information displayed on the withdrawal display 83.

A withdrawal communicating step S96 is performed for communicating withdrawal operation information, including the name of the withdrawn medicine and the number thereof, to the dispensation managing section 11, while making the information correspond to the ID code of the medicine container 92.

As thus described, in the dispensation operation, when a cancellation of the prescription direction occurs, the medicine container 92 corresponding to the prescription code of the prescription direction cancelled is specified. The medicine withdrawing section 17 determines whether or not a medicine in the medicine container 92 corresponds to a withdrawal medicine, based upon the management information on the medicine container 92. Further, the medicine withdrawal information on the medicine container 92 is showed, based upon the management information.

The medicine withdrawal information includes the result of determination as to whether or not each detected medicine is a withdrawal medicine. As thus described, the medicine withdrawal section 17 shows the withdrawal medicine, then the cancelled medicine can be reused.

It should be noted that the withdrawal operation step S03 has the cancelled medicine determining step S94. The cancelled medicine determining step S94 as the withdrawal process, for example, determines whether or not a medicine in the cancelled medicine container 92 corresponds to a withdrawal medicine, based upon the management information and the cancellation information on the medicine container 92.

Some medicines cannot be accurately determined, just based upon the cancellation information, as to whether or not the medicine corresponds to a reusable withdrawal medicine. The management information is thus used to determine whether or not the cancelled medicine is reusable. It is possible to perform an accurate determination on the cancelled medicine, on which the determination is difficult just based upon the cancellation information.

Further, the withdrawal operation step S03 has the cancellation presenting step S93 which, for example, presents the cancellation information on the cancelled medicine container 92, as the withdrawal process.

In a place where the withdrawal operation is performed, the ID code of the cancelled medicine container 92 is obtained, and the cancellation information linked to the ID code of the medicine container 92 is presented. This allows the withdrawal person to know the cancellation information on the medicine container 92, thereby to accurately withdraw the medicine.

Further, the withdrawal operation step S03 has a medicine information detecting step of, for example, detecting medicine information on a medicine as the withdrawal process. Moreover, the withdrawal operation step S03 has the cancelled medicine determining step S94 for determining whether or not each detected medicine corresponds to a withdrawal medicine. Furthermore, the withdrawal operation step S03 has a medicine withdrawal information presenting step of showing a withdrawal medicine determination result as the medicine withdrawal information.

As thus described, the withdrawal operation step S03 has the medicine information detecting step, the cancelled medicine determining step and the medicine withdrawal information presenting step, whereby the withdrawal medicine determination result with respect to each cancelled medicine can be showed to the withdrawal person. Therefore, the medicine withdrawal is accurately performed without dealing a wrong medicine. Further, the cancelled medicine determining step S94 determines whether a medicine in the cancelled medicine container 92 corresponds to a withdrawal medicine, corresponds to a disposal medicine, or corresponds to a non-cancelled medicine, from the management information of the medicine container 92.

In the case of occurrence of a cancellation, it is possible to sort at once each of the medicines in the medicine container 92 to one among a withdrawal medicine, a disposal medicine and a non-cancelled medicine.

Further, the cancelled medicine determining step S94 determines whether or not a cancelled medicine in the medicine container 92 corresponds to a withdrawal medicine, from the dispensation progress information in the management information on the medicine container 92. Since it is determined whether or not the medicine corresponds to a withdrawal medicine, based upon the progress status of the dispensation, more accurate determination can be performed.

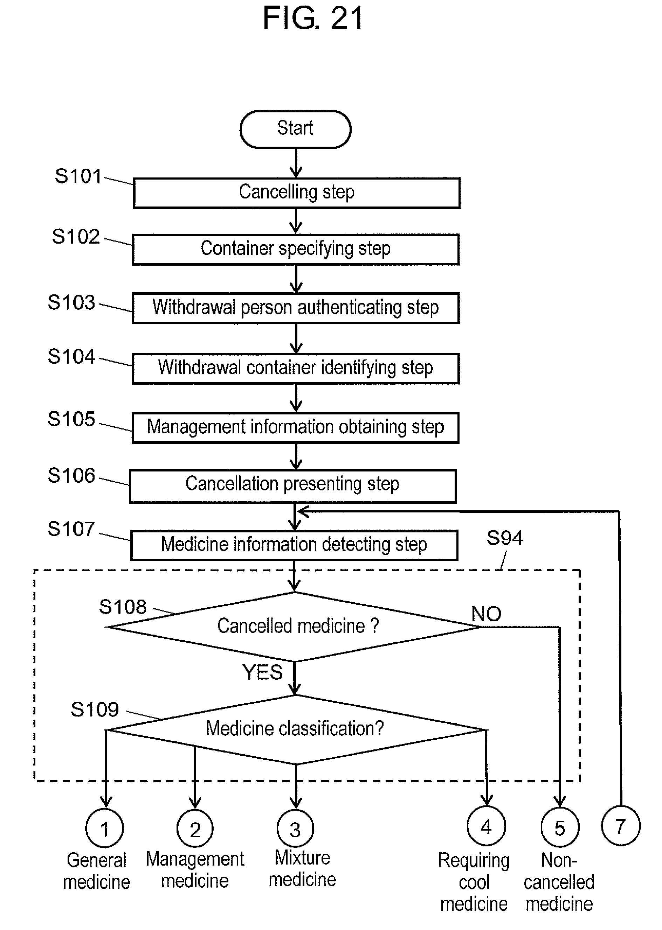

FIGS. 21 to 27 are detailed flowcharts for the withdrawal operation. Next, details of the medicine withdrawal operation are described with reference to these flowcharts.

When the doctor judges to change or cancel a prescription with a prescription direction, the doctor generates a cancellation of the prescription through the progress inquiring section 22 or the like. A cancelling step S101 is performed for communicating to the dispensation managing section 11 that the prescription direction has been cancelled.

First, the medicine withdrawing section 17 receives the cancellation information. Thereafter, the medicine withdrawing section 17 transmits the cancellation information to the dispensation managing section 11. The cancellation information includes such contents of the cancellation that the whole or parts of the medicines in the dispensation direction information are cancelled.

In the dispensation managing section 11, a container specifying step S102 is then performed for specifying the ID code of the medicine container 92 corresponding to the prescription code of the prescription direction cancelled. The cancellation information is recorded in the management information by linking to the ID code of the medicine container 92.

The cancelled medicine container 92 is carried to the dispensation operating section, for example, the medicine procuring section 15. The medicine procuring section 15 detects the ID code of the medicine container 92, and makes an inquiry to the dispensation managing section 11, to obtain the prescription direction corresponding to the ID code.

At this time, the dispensation managing section 11 checks the presence or absence of the cancellation information with respect to the medicine container 92, and when the cancellation information is present, the dispensation managing section 11 transmits to the medicine procuring section 15 that the cancellation of the medicine container 92 has occurred. The medicine procuring section 15 then shows that the cancellation of the medicine container 92 has occurred.

The dispensation operator is informed that the cancellation has occurred, and carries the medicine container 92 to the medicine withdrawing section 17. When the cancelled medicine container 92 is carried into the medicine withdrawing section 17, the medicine withdrawal operation is started. First, a withdrawal person authenticating step S103 is performed for authenticating a withdrawal person with the person authentication device 82.

The person authentication device 82 compares operator information of the dispensation operator with the register information, to check whether or not the operator is a dispensation operator permitted for a medicine withdrawal, namely a withdrawal person. When having been permitted for the operation, the operator is authenticated as a withdrawal person.

The RFID tag 93 of the medicine container 92 carried into the section is read with the RFID reader 81a. That is to say, a withdrawal container identifying step S 104 is performed for identifying the ID code of the medicine container 92.

Next, an inquiry is made to the dispensation managing section 11, to check the presence or absence of the cancellation information on the detected ID code of the medicine container 92. When the cancellation information is present, a management information obtaining step S105 is performed for obtaining the management information on the medicine container 92 which includes the cancellation information.

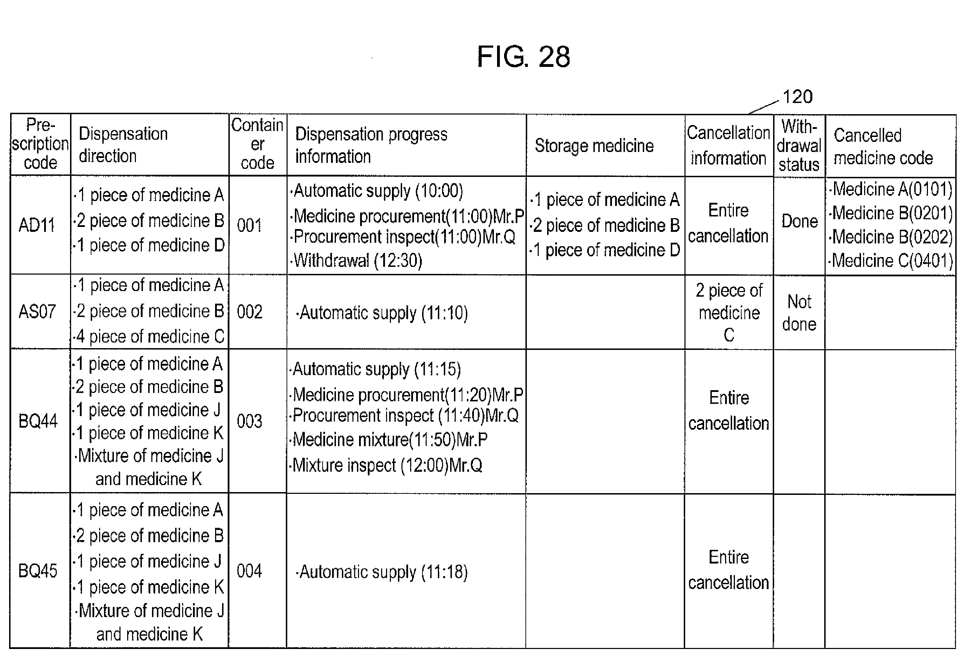

FIG. 28 shows a medicine management table 120, in which the management information is put down. The prescription code, the dispensation direction information, the container code, the dispensation progress information, the storage medicine information, the cancellation information, the withdrawal status, and the cancelled medicine code are recorded in the medicine management table 120, as the management information. The dispensation progress information has records of an operation time and an operator of the dispensation operation. When the dispensation operation is performed, the operation time and the operator are determined in accordance with the supply information, the procurement information and the mixture information as the dispensation information.

The storage medicine means the medicines collected into the medicine container 92. The medicines collected into the medicine container 92 are calculated from the supply information, the procurement information and the mixture information as the dispensation information.

Returning to the flowchart of FIG. 21, a description is given. After the management information including the cancellation information has been obtained, a cancellation presenting step S106 is performed for showing the cancellation information of the medicine container 92 on the withdrawal display 83.

After the cancellation information has been presented, the withdrawal person picks up one of the medicines in the medicine container 92 and reads the bar code of the picked medicine with the bar code reader 88. A medicine information detecting step S107 is then performed.

The medicine withdrawing section 17 identifies the medicine name of the detected medicine from the medicine information. It is then determined whether or not the medicine name is a name of the cancelled medicines (the step S108).

When the medicine name is the name of the cancelled medicine, a classification of the medicine types is performed according to the medicine name of the detected medicine (the step S109). The medicine types mentioned, herein, are groups according to the medicine management or groups according to the dispensation method. Specifically, medicines are classified into: a general medicine; a management medicine whose treatment is required to be managed by a pharmacist or the like; a mixture medicine obtained by mixing a plurality of medicines; and the requiring cool medicine whose temperature is required to be managed cool. Although a mixture medicine can also be taken as a general medicine as well as a management medicine, a general medicine and a management medicine aimed to be mixed are taken as a mixture medicine.

The classification of the medicine types is determined based upon which medicine group the medicine name belongs to. Next, the withdrawal operation on each classified medicine type is described.

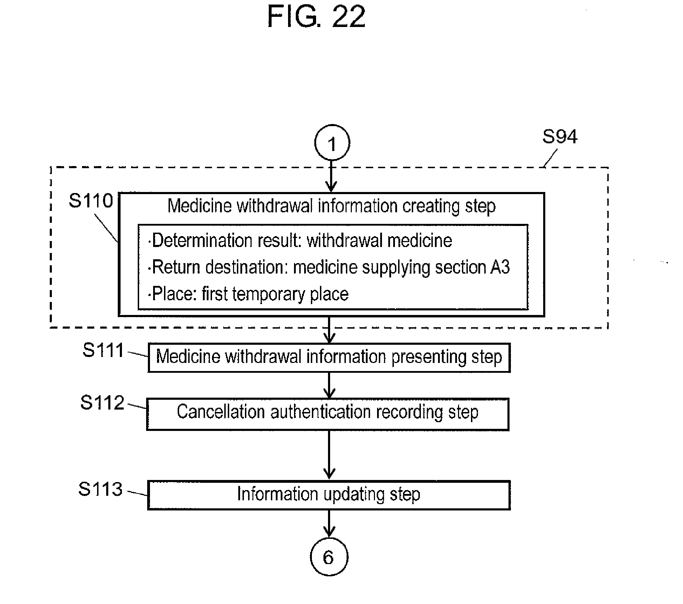

First, the case of the classification as a general medicine is described with reference to the second flowchart of FIG. 22. When the detected medicine is classified as a general medicine, a medicine withdrawal information creating step S110 is first performed.

Since a general medicine corresponds to a reusable withdrawal medicine, the determination result is the withdrawal medicine. For example, when a medicine A as a general medicine is detected, medicine withdrawal information as the withdrawal medicine is created with the general medicine.

As the medicine withdrawal information created, the medicine withdrawal information has: a cancelled medicine determination result; a return destination of the cancelled medicine; a place as a temporary place for the cancelled medicine; and the cancelled medicine code.

Part of the medicine withdrawal information is shown in the step S110 of FIG. 22. The determination result being the withdrawal medicine means that the cancelled medicine corresponds to a withdrawal medicine. The return destination being medicine supplying section A3 means that the withdrawal medicine is returned to the third case of the medicine supplying section 14. The place means a container which holds temporarily withdrawal medicines therein or a disposal box into which a disposal medicine is discarded, in the medicine withdrawing section 17. The first temporary place means a temporary placement container for holding therein withdrawal medicines to be returned to the medicine supplying section 14. The cancelled medicine code is the identification number for managing the cancelled medicine.

In the withdrawal display 83, a medicine withdrawal information presenting step S111 is performed for showing the medicine withdrawal information on the detected medicine to the withdrawal person.

Next, a cancellation authentication recording step S112 is performed for recording the cancel authentication information indicating that the medicine is a cancelled medicine. Specifically, a label is printed with the medicine withdrawal information as the cancellation authentication information. Subsequently, an information updating step S113 is performed for updating the management information and the cancelled medicine information of the dispensation managing section 11.

At this time, the withdrawal person sticks the printed label to the medicine A and puts the medicine A into the first temporary place in accordance with the medicine withdrawal information presented. Sticking the label with the medicine withdrawal information to the medicine means recording the cancellation authentication information on the medicine which is determined as a cancelled medicine in the withdrawal operation.

In the case of reusing the medicine as a withdrawal medicine, looking at the seal label stuck to the medicine can facilitate the determination of whether or not the medicine is a normal medicine or a withdrawal medicine.

In addition, the cancellation authentication information may not be recorded on the label, but the information may be recorded in an RFID of the medicine as a withdrawal medicine. Further, direct printing may be performed on the surface of the medicine. The surface of the medicine means the surfaces of a medicine glass bottle, a medicine bag, or the like. Further, in place of the medicine withdrawal information, a mark may simply be printed to indicate that the medicine is a cancelled medicine.

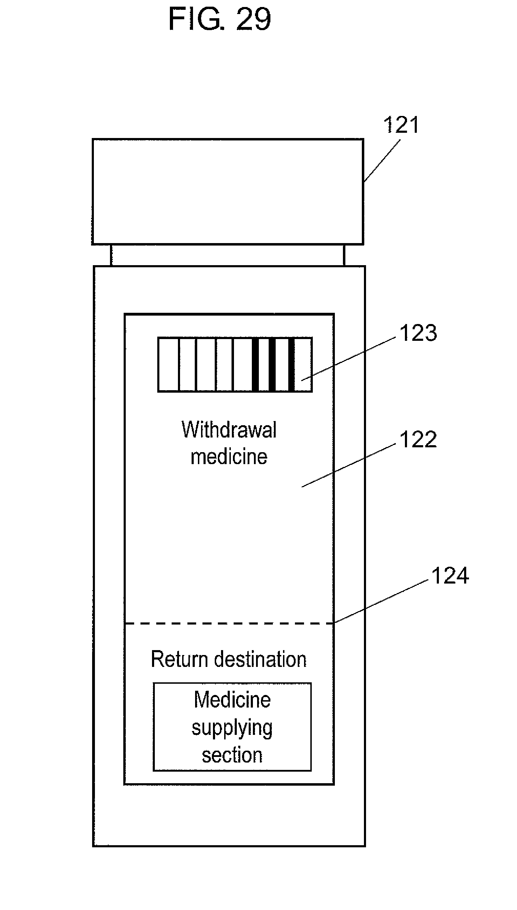

FIG. 29 is a front view of a cancelled medicine stuck with a label. On a label 122 stuck to a withdrawal medicine 121 as a cancelled medicine, a variety of information is recorded. The cancelled medicine code is recorded as a bar code 123. Further, the return destination is printed below a cutoff line 124 of the label 122. It should be noted that the medicine withdrawal information described above is for the medicine A, and hence, for example, the return destination of the medicine B as a general medicine, is a different place.

Moreover, in an information updating step S113, the cancelled medicine information included in the management information is updated.

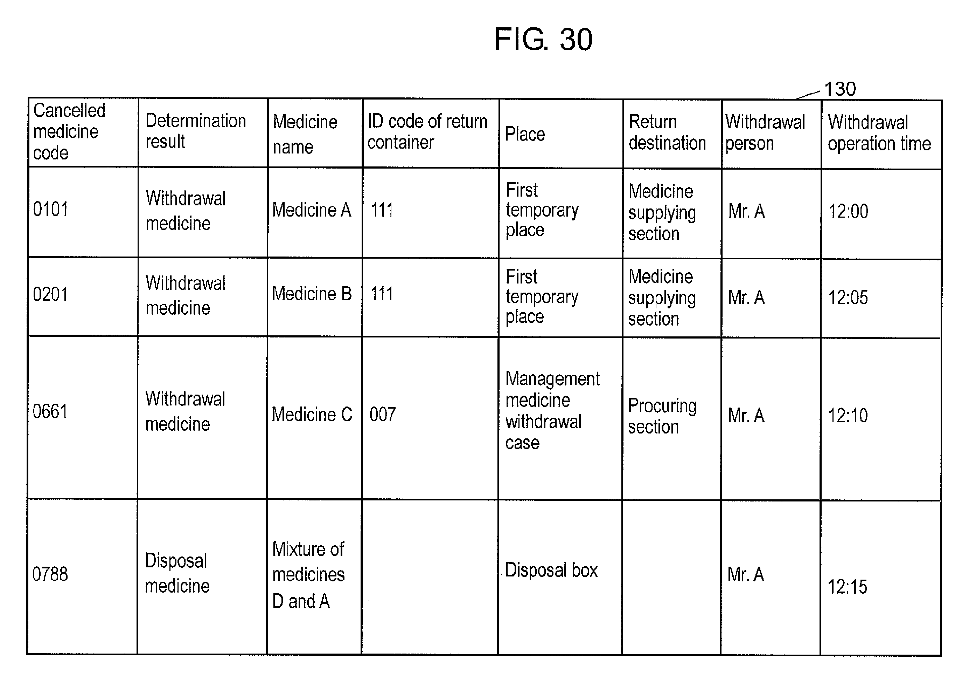

FIG. 30 is a diagram showing a cancelled medicine management table for the cancelled medicine information. The cancelled medicine code, the determination result, the medicine name, the ID code of the return container, the place, the return destination, the withdrawal person, the cancelled medicine management information such as the withdrawal operation time are recorded in the cancelled medicine management table 130. Although not put down in FIG. 30, the dispensation progress information may be included.

As thus described, the withdrawal operation step S03 has the cancellation authentication recording step S112 for recording the cancellation authentication recording information that indicates a cancelled medicine, determined as a withdrawal medicine or a disposal medicine.

Recording the cancellation authentication information on each cancelled medicine can facilitate the identification of the cancelled medicine even when new medicines and cancelled medicines are intermingled. Therefore, the cancellation authentication information may have letters "withdrawal medicine" or "disposal medicine". This makes it easy to identify a cancelled medicine at a glance.

Further, in the cancellation authentication recording step S112, the cancellation authentication information including the cancelled medicine code may be recorded on the withdrawal medicine. As shown in FIG. 28, the cancelled medicine code is recorded as the management information in the medicine management table 120. By recording of this cancelled medicine code, the history of the withdrawal medicine can be seen from the cancelled medicine code.

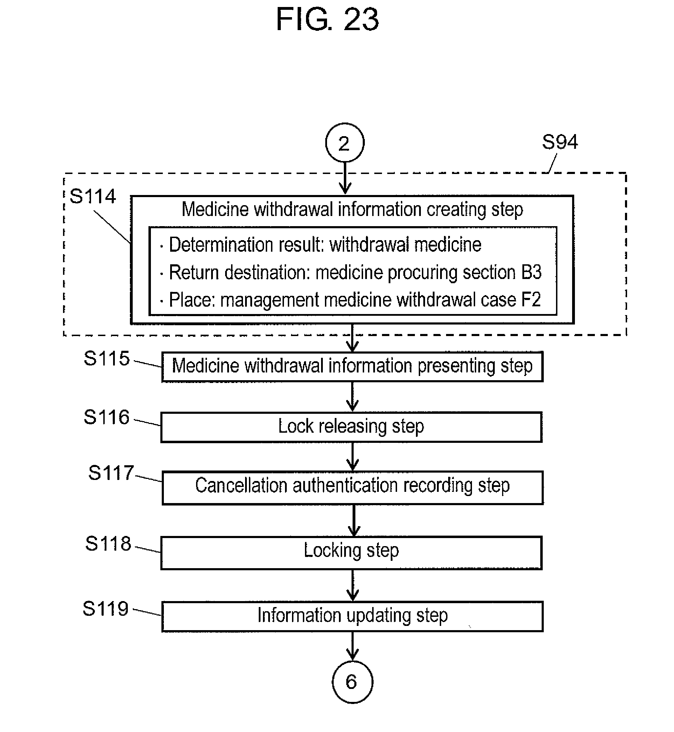

Next, the case of classification as a management medicine is described with reference to the third flowchart of FIG. 23. When the detected medicine is classified as a management medicine, a medicine withdrawal information creating step S114 is performed. Since a management medicine corresponds to a reusable withdrawal medicine, the determination result is a withdrawal medicine. Therefore, the medicine withdrawal information as a withdrawal medicine is created for the management medicine.

The determination result being a withdrawal medicine, shown in the step S114 of FIG. 23, means that a medicine D as a management medicine corresponds to a withdrawal medicine. The return destination being the medicine procuring section 15, B3 means that the medicine is returned to the third case of the management medicine shelf case 99 of the medicine procuring section 15. The place being the management medicine withdrawal case F2 means that the withdrawal medicine is temporarily held in the second case of the management medicine withdrawal case 111. At this time, although not shown in the figure, the cancelled medicine code is also created as the medicine withdrawal information.

Each case of the management medicine withdrawal case 111 is provided with a locking mechanism so as to hold the management medicine therein. A medicine withdrawal information presenting step S115 is performed by the withdrawal display 83 for presenting the medicine withdrawal information to the withdrawal person. Next, a lock releasing step S116 is performed for releasing the lock of the second case of the management medicine withdrawal case 111. Subsequently, a cancellation authentication recording step S117 is performed for printing a label recorded with the medicine withdrawal information.

At this time, the withdrawal person sticks the printed label to the medicine D and puts the medicine D into the second case of the management medicine withdrawal case 111 in accordance with the medicine withdrawal information showed.

The withdrawal person presses a locking button (not shown) of the medicine withdrawing section 17 so as to lock the management medicine withdrawal case 111. Thereupon, a locking step S118 is performed for locking the management storeroom of the medicine withdrawing section 17. An information updating step S119 is then performed for updating the cancelled medicine information and the management information of the dispensation managing section 11.

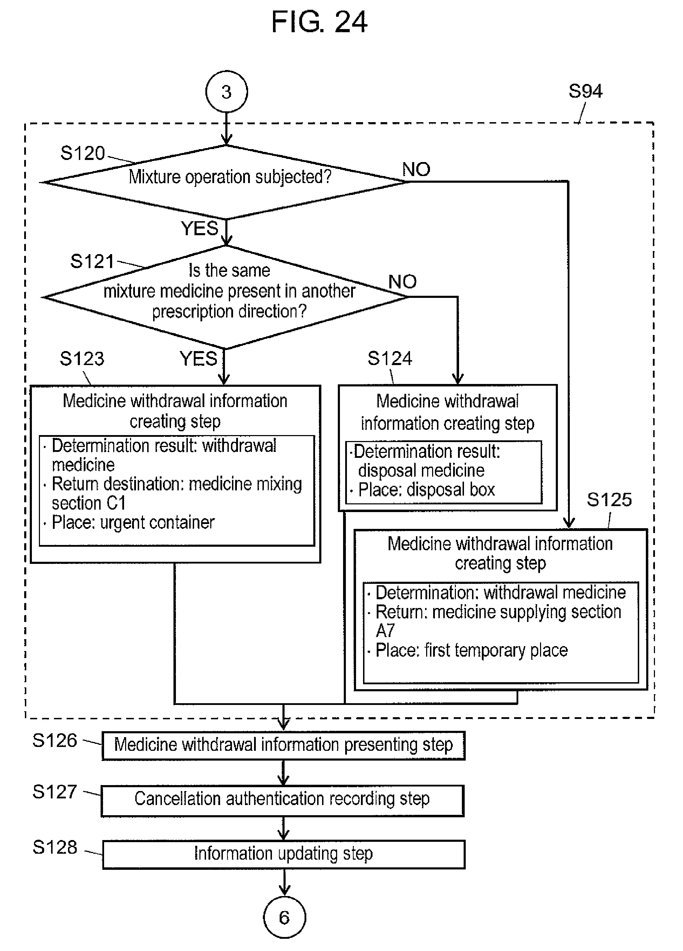

Next, the case of classification as a mixture medicine is described with reference to the fourth flowchart of FIG. 24. In the case of a mixture medicine being present in the medicines of the medicine container 92, a cancelled medicine determining step S94 determines that the mixture medicine corresponds to a withdrawal medicine when the dispensation progress information as the management information of the medicine container 92 indicates pre-mixture.

In other words, when the detected medicine is classified as a mixture medicine, it is determined whether or not the mixture medicine has already been subjected to the mixing operation (the step S120).

As in the medicine management table 120 shown in FIG. 28, the dispensation progress information is included in the management information, and the history of the dispensation operation is recorded. For example, medicines of the prescription code BQ44 in the medicine container 92 has already been subjected to a medicine mixture according to the dispensation progress information.

When the medicine is determined as post-mixture, the management information of the dispensation managing section 11 is searched on whether or not there is a prescription direction including the same mixture medicine before the mixing operation. It is then determined whether or not the same mixture medicine is present in another prescription direction (the step S121).

The post-mixture medicine cannot be used as a medicine unless used within a certain period of time. Described herein is a method for having an already mixed medicine used in a different place when another prescription direction needs the same mixture medicine inside the hospital.

When the same mixture medicine is needed in another prescription direction, a medicine withdrawal information creating step S123 is performed for creating the medicine withdrawal information as a reusable withdrawal medicine.

Part of the medicine withdrawal information is shown in the step S123 of FIG. 24. The medicine mixing section C1 means that the medicine is returned to the first case of the medicine mixing section 16. The place being the urgent container means the container for urgently delivering a withdrawal medicine to the dispensation operating section. The medicine in the urgent container is carried to the destination upon the completion of the withdrawal operation on one medicine container 92.

Further, in the case of the same mixture medicine being not present in another prescription direction in the step S121, a medicine withdrawal information creating step S124 is performed for sorting the cancelled medicine to a disposal medicine. Such a post-mixture medicine does not correspond to a withdrawal medicine, but corresponds to a disposal medicine. The post-mixture medicine becomes more difficult to reuse with the passage of time. The already mixed medicine, for which the same prescription direction has not been generated, is taken as a disposal medicine.

In such a case, the medicine withdrawal information is created on the post-mixture medicine as a disposal medicine. Part of the medicine withdrawal information is shown in the step S125 of FIG. 24. The determination result is the disposal medicine, and the place is the disposal box into which a disposal medicine is discarded.

Further, when it is determined in the step S120 that the medicine is before being subjected to the mixing operation, a medicine withdrawal information creating step S125 is performed on the medicine as a reusable withdrawal medicine. In such a case, the medicine withdrawal information is created on the pre-mixture medicine as a withdrawal medicine. Part of the medicine withdrawal information is shown in the step S125 of FIG. 24. At this time, the determination result is a withdrawal medicine, the return destination is the medicine supplying section A7, and the place is the first temporary place.

The pre-mixture medicine at this time is returned to the medicine supplying section 14 since being a general medicine, but in the case of the pre-mixture medicine being a management medicine, the return destination is the medicine procuring section 15.

As thus described, a mixture medicine is sorted according to the conditions of the dispensation progress information in the management information, and the medicine withdrawal information creating step is performed after the determination of whether or not the mixture medicine corresponds to a withdrawal medicine.

Upon the completion of the medicine withdrawal information creating steps S123, S124 and S125, a medicine withdrawal information presenting step S126 is performed for presenting the medicine withdrawal information to the withdrawal person with the withdrawal display 83. Next, a cancellation authentication recording step S127 is performed for printing the label, which is recorded with the medicine withdrawal information. Subsequently, an information updating step S128 is performed for updating the cancelled medicine information and the management information of the dispensation managing section 11.

At this time, the withdrawal person sticks the printed label to the medicine, and places the medicine on the designated place in accordance with the medicine withdrawal information displayed. It is to be noted that the printed label is stuck even to a disposal medicine, so as to prevent the disposal medicine from being intermingled with a withdrawal medicine.

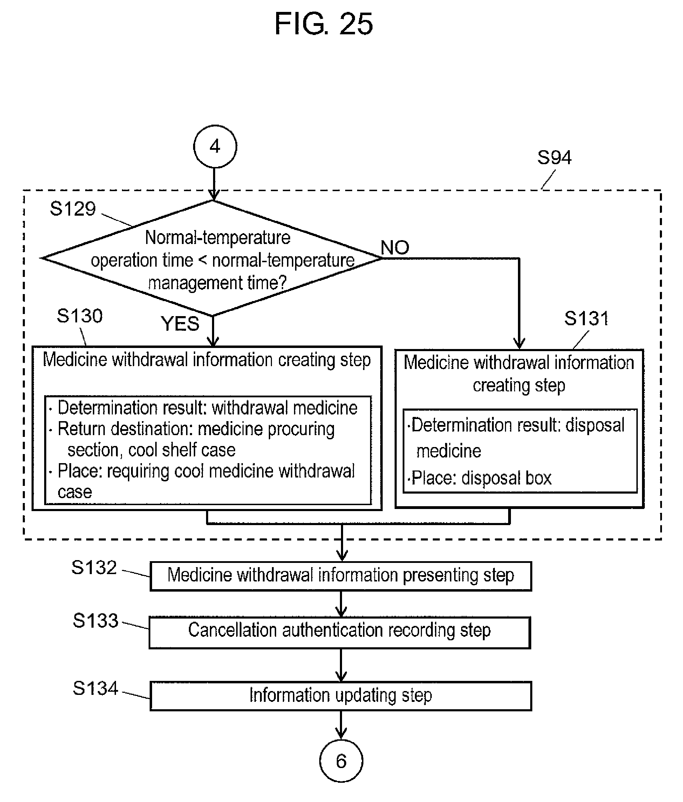

Next, the case of classification as the requiring cool medicine is described with reference to the fifth flowchart of FIG. 25. In the case of a requiring cool medicine being present in the medicines of the medicine container 92, the cancelled medicine determining step S94 calculates a normal-temperature operation time as a stay time for the requiring cool medicine after being taken out to a normal-temperature environment, from the procurement time of the dispensation progress information in the management information of the medicine container 92.

When the normal-temperature operation time is shorter than a normal-temperature management time, it is determined that the requiring cool medicine corresponds to a withdrawal medicine. In other words, when the detected medicine is classified as the requiring cool medicine, the normal-temperature operation time as the stay time for the requiring cool medicine after being taken out to the normal-temperature environment is calculated. It is then determined whether or not the normal-temperature operation time is shorter than the normal-temperature management time (the step S129).

As shown in FIG. 28, the dispensation progress information is included in the management information, and the time when the dispensation operation is performed is recorded. The time from this medicine procurement time to the time when the medicine is detected in the medicine withdrawing section 17 is calculated as the normal-temperature operation time. Then, the normal-temperature operation time is compared with the normal-temperature management time, which is the time when the medicine is usable even in the normal-temperature environment.

When the normal-temperature operation time is shorter than the normal-temperature management time, a medicine withdrawal information creating step S130 is performed for creating the medicine withdrawal information of the requiring cool medicine as a reusable withdrawal medicine.

Part of the medicine withdrawal information is shown in the step S130 of FIG. 25. The determination result is the withdrawal medicine, the return destination is the cool shelf case 100 of the medicine procuring section 15, and the place is the requiring cool medicine withdrawal case 112.

Further, in the step S129, when the normal-temperature operation time is not shorter than the normal-temperature management time, a medicine withdrawal information creating step S131 is performed for creating the medicine withdrawal information of the requiring cool medicine as a disposal medicine.

Such a requiring cool medicine does not correspond to a withdrawal medicine, and corresponds to a disposal medicine. Part of the medicine withdrawal information is shown in the step S131 of FIG. 25. At this time, the determination result is the disposal medicine, and the place is the disposal box.

Upon completion of the medicine withdrawal information creating steps S130 and S131, a medicine withdrawal information presenting step S132 is performed for presenting the medicine withdrawal information to the withdrawal person with the withdrawal display 83. A cancellation authentication recording step S133 is performed for printing the label recorded with the operator information. An information updating step S134 is then performed for updating the cancelled medicine information and the management information of the dispensation managing section 11. At this time, the withdrawal person sticks the printed label to the medicine, and places the medicine in the designated place in accordance with the medicine withdrawal information presented.

Next, the case of a non-cancelled medicine is described. As shown in FIG. 21, before a classification of the medicine types has been performed (the step S109), the cancelled medicine determination (the step S108) is performed.

There is a cancellation of a prescription direction which cancels part of the medicines, and thereat, a non-cancelled medicine which is not a cancelled medicine is present in the medicine container 92. Such a non-cancelled medicine is returned to the medicine container 92 after removal of cancelled medicines.

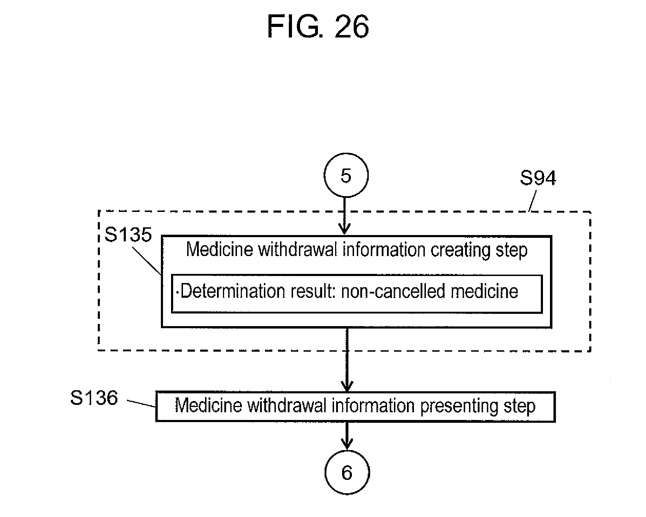

As shown in FIG. 26, when it is determined in the cancelled medicine determination (the step S108) that the medicine is not a cancelled medicine, a medicine withdrawal information creating step S135 is performed for creating the medicine withdrawal information on the non-cancelled medicine. The medicine withdrawal information, indicating the determination result of the detected medicine being a non-cancelled medicine, is created. A medicine withdrawal information presenting step S136 is then performed for presenting the medicine withdrawal information. The withdrawal person is informed that the detected medicine is a non-cancelled medicine.

The medicine withdrawal information created in the step S130 presents a message to the withdrawal person. However, the management information is not updated, and a label is not printed.

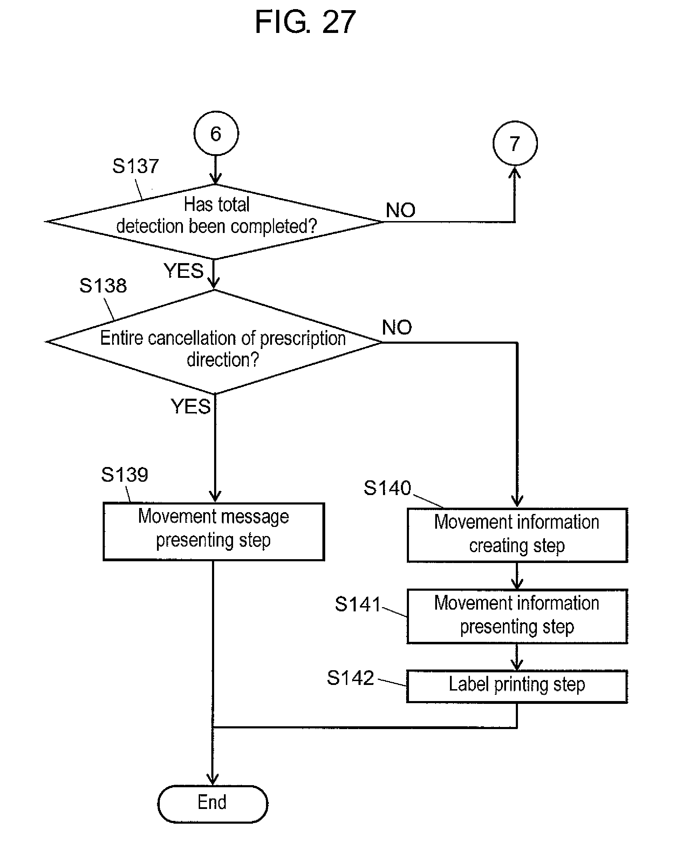

Upon completion of one among the step S113, the step S119, the step S128, the step S134 and the step S136, as shown in the seventh flowchart of FIG. 27, it is determined whether or not total detection of the medicines in the medicine container 92 has been completed (the step S137).

When all of the medicines as the storage medicines in the management information have not been detected, the process returns to the step S107.

Upon completion of detecting all of the medicines as the storage medicines in the management information, it is determined whether or not the cancellation information indicates an entire cancellation (the step S138).

When the information indicates the entire cancellation, the medicine container 92 is returned to a container place. Thereat, a movement message presenting step S139 is performed for presenting a message of the movement to the container place in the withdrawal display 83.

When the information does not indicate the entire cancellation, a non-cancelled medicine is returned to the medicine container 92. Then, the medicine container 92 needs to be returned to the dispensation operating section. Therefore, a movement information creating step S140 is performed for directing the movement destination of the medicine container 92. Next, a movement information presenting step S141 is performed for presenting the movement information on the withdrawal display 83. Subsequently, a label printing step S142 is performed for printing a label with the movement information of the container.

In accordance with this movement information, the withdrawal person moves the medicine container 92 with the non-cancelled medicine put therein. In such a manner, the medicine withdrawal operation is performed. As thus described, the medicine management system 12 can manage the dispensation information and the progress of each dispensation operation with respect to each patient. The system can then perform accurate dispensation for each patient. Thereby, even in the case of occurrence of cancelled medicines, it is possible to determine a reusable withdrawal medicine since the dispensation information and the progress management of the dispensation operation is managed with accuracy.

It should be noted that the withdrawal operation may not be performed in the medicine withdrawing section 17, but be performed in each dispensation operating section. In the case of performing the withdrawal operation in each dispensation operating section, a mode of the dispensation operating section is changed from a normal mode to a withdrawal mode, for performing the withdrawal operation.

Further, although the withdrawal-medicine determination is performed upon a detection of a medicine with the bar code reader 88, the withdrawal-medicine determination may be performed when the medicine container 92 is placed in the medicine container placement section 81. The withdrawal display 83 may be made to display, as the medicine withdrawal information, the determination result of the withdrawal medicine in addition to the cancellation information. The withdrawal person can see which a withdrawal medicine is in the medicine container 92 before detecting the medicine with the bar code reader 88.

When the medicine withdrawal operation is performed and the withdrawal medicines are accumulated in the place, the dispensation operator carries the management medicine withdrawal case 111 or the like to each dispensation operating section, and the medicine return operation is performed in each dispensation operation section.

Next a return operation for returning a withdrawal medicine to each dispensation operating section is described. As a specific example of the return operation, an operation to return a management medicine to the medicine procuring section 15 is described.

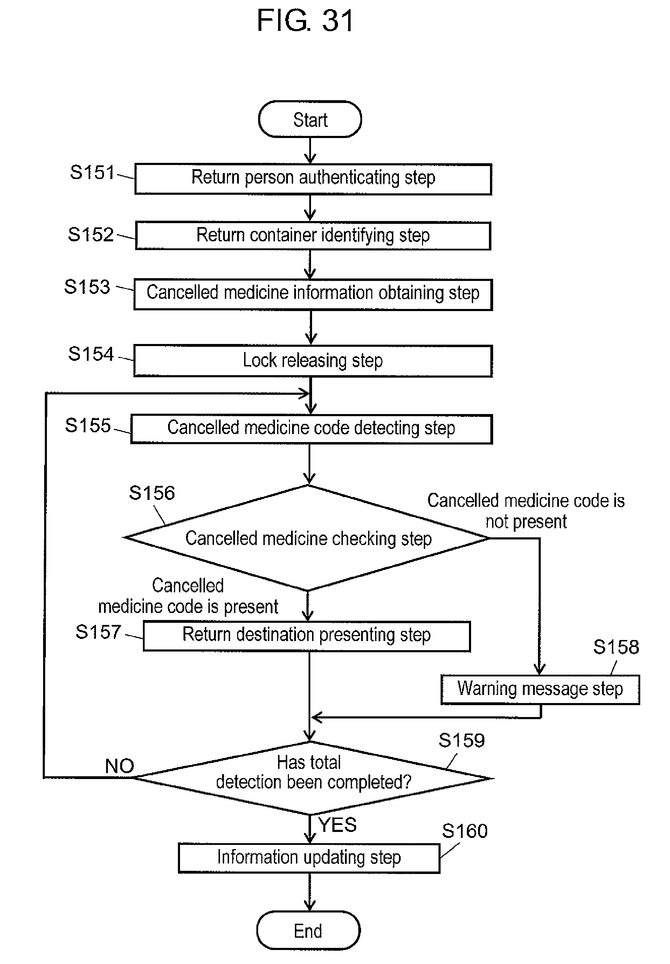

FIG. 31 is a flowchart for the return operation performed in the medicine procuring section 15. A dispensation operator carries one of the management medicine shelf cases 111 of the medicine withdrawing section 17 as a return container to the medicine procuring section 15. The dispensation operator switches the medicine procuring section 15 from a medicine procurement and procurement inspection mode to a return operation mode. The return operation is then performed in the medicine procuring section 15.

First, a return person authenticating step S151 is performed. The person authentication device 42 compares the dispensation operator information of the dispensation operator with the register information, to check whether or not the dispensation operator is a dispensation operator permitted for the return operation, namely a return person. When a non-registered dispensation operator performs the authenticating operation, letters "not-permitted" are displayed on the procurement display 43. When a registered dispensation operator performs the authenticating operation, letters "permitted" are displayed on the procurement display 43, and the return operation is permitted.

That is, in the return person authenticating step S151, the operator information of the return person is obtained, and when the return person is qualified to return a medicine, the return person is permitted to return the medicine to the dispensation operating section.

In such a manner, when a medicine determined as a withdrawal medicine is to be returned to the dispensation operating section, the operator information of the return operator is obtained, and when the return person is qualified to return the medicine, the medicine is returned to the dispensation operating section.

Subsequently, a return container identifying step S152 is performed. After the ID code of the return container has been identified, a cancelled medicine information obtaining step S153 is performed for obtaining the cancelled medicine information of the withdrawal medicines in the return container from the dispensation managing section 11.

The cancelled medicine information includes the ID code of the return container. The cancelled medicine information of the withdrawal medicines in the return container is obtained according to the ID code of the return container. A lock releasing step S154 is then performed for releasing the lock of the management medicine shelf case 99 of the medicine shelf. This makes it possible to return the management medicine to the management medicine shelf case 99.

Herein, the return person picks up one by one the management medicines in the management container for delivery, and detects the label 122 of the withdrawal medicine 121 with the bar code reader 45. In the medicine procuring section 15, a cancelled medicine code detecting step S155 is performed for detecting the cancelled medicine code from the label 122. It is then determined whether or not the cancelled medicine information corresponding to the cancelled medicine code detected is present in the cancelled medicine information obtained in the step S153. In other words, a cancelled medicine checking step S156 is performed.

When the cancelled medicine code is present in the cancelled medicine information, a return destination presenting step S157 is performed for presenting a return destination. For example, letters "return to the second case of the management medicine shelf" are showed on the procurement display 43. When there is no cancelled medicine code present in the cancelled medicine information, it is determined that a medicine other than the withdrawal medicines has been brought in. A warning message step S158 is then performed for showing a warning message indicating that the medicine is a nonreturnable medicine.

As thus described, only the medicine determined as a withdrawal medicine is made returnable to the dispensation operating section.

To that end, the withdrawal operation step S20 has the cancellation authentication recording steps S112, S117, S127 and S133 for recording the cancellation authentication information including the cancelled medicine codes of the withdrawal medicines, while recording the cancelled medicine codes into the dispensation managing section 11.

Then, the return operation step has the cancelled medicine checking step S156 for performing the following determination at the time of returning a medicine as a withdrawal medicine to the dispensation operating section. The cancelled medicine checking step S156 determines whether or not the cancelled medicine code of the cancellation authentication information, detected in the dispensation operating section, is recorded in the dispensation managing section 11.

When the cancelled medicine code is recorded in the dispensation managing section, it means that an appropriate medicine return has been made. When there is no cancelled medicine code in the dispensation managing section, it means that an inappropriate medicine return has been made.

Further, at the time of returning the withdrawal medicine 121, the return person cuts out the seal strip, recorded with the return destination, along the cutoff line 124 shown in FIG. 29. In the case of using a withdrawal medicine, the dispensation operator can see it at a glance in the medicine procuring section 15, that the medicine is a withdrawal medicine rather than a normal medicine.

Upon completion of the step S157 or the step S158, the step S159 checks whether or not total detection of the medicines in the management container for delivery has been completed.

When the detection of all the medicines has not been completed, the process returns to the step S155, and when the detection of all the medicines has been completed, an information updating step S160 is performed for updating the cancelled medicine information of the dispensation managing section 11. As an update of the cancelled medicine information, the withdrawal operation time and the withdrawal person are recorded into the cancelled medicine information.

As thus described, the medicine management system 12 specifies the medicine container to make an accurate cancellation, and it is possible to reduce withdrawal errors at the time of the cancellation. Further, the medicine management system 12 can directly procure the medicines in the medicine container 92 used for a medicine delivery, thus eliminating the operation of transferring medicines. Moreover, it is possible to manage each dispensation operation with respect to each medicine container 92. In other words, it is possible to present supplementary medicine information corresponding to the medicine container 92, so as to reduce procurement errors. Further, it is possible to present procurement medicine information corresponding to the medicine container 92, so as to reduce procurement inspection errors.

Thereat, the medicine management system 12 has the prescription generating section 21, the medicine supplying section 14, the medicine procuring section 15, the medicine mixing section 16, the medicine withdrawing section 17, and the medicine delivering section 18.

A medicine management is performed by the following method using the medicine management system 12. The prescription generating section 21 performs the prescription generating step S31 for generating a prescription direction including a prescription code and dispensation direction information.

The medicine supplying section 14 performs the medicine supplying step S12. The medicine supplying step S12 has the automatic supply step S41 and the supply communicating step S42.

The automatic supply step S41 uses the medicine container 92 having the ID code, to automatically supply medicines to the medicine container 92 in accordance with the prescription direction.

The supply communicating step S42 makes the ID code of the medicine container 92 and the prescription code of the prescription direction have a corresponding relation to each other, and communicates to the dispensation managing section 11, the corresponding relation and supply information regarding the medicines inserted into the medicine container.

In the medicine procuring section 15, the medicine procuring step S14 and the procurement inspecting step S15 are performed. The medicine procuring step S14 has the procurement person authenticating step S51, the procurement container identifying step S52, the procurement presenting step S53, and the procurement communicating step S54. The procurement inspecting step S15 has the procurement inspector authenticating step S55, the procurement inspection presenting step S56, and the procurement inspection communicating step S57.

The procurement person authenticating step S51 compares an authenticated procurement person with register information, to make a check. The procurement container identifying step S52 identifies the ID code of the medicine container 92. The procurement presenting step S53 presents supplementary medicine information on the medicine container 92, based upon management information of the dispensation managing section corresponding to the ID code of the medicine container. The procurement communicating step S54 communicates procurement information, including procurement person information, to the dispensation managing section 11, while making the procurement information correspond to the ID code of the medicine container 92.

The procurement inspector authenticating step S55 compares an authenticated procurement inspector with register information, to make a check. The procurement inspection presenting step S56 presents information of the medicines procured in the medicine container 92, based upon the management information of the dispensation managing section 11 corresponding to the ID code of the medicine container.

The procurement inspection communicating step S57 communicates procurement inspection information, including procurement inspector information, to the dispensation managing section 11, while making the procurement inspection information correspond to the ID code of medicine container 92.

In the medicine mixing section 16, the medicine mixing step S17 and the mixture inspecting step S18 are performed. The medicine mixing step S17 has the mixing person authenticating step S61, the mixture container identifying step S62, the mixture presenting step S63, and the mixture communicating step S64.

The mixture inspecting step S18 has the mixing inspector authenticating step S65, the mixture inspection presenting step S66, and the mixture inspection communicating step S67.

The mixing person authenticating step S61 compares the authenticated mixing person with register information, to make a check. The mixture container identifying step S62 obtains the ID code of the medicine container 92. The mixture presenting step S63 presents mixture medicine information on the medicine container 92, based upon the management information of the dispensation managing section corresponding to the ID code of the medicine container 92. The mixture communicating step S64 communicates mixture information, including mixing person information, to the dispensation managing section 11, while making the mixture information correspond to the ID code of the medicine container 92.

The mixture inspector authenticating step S65 compares an authenticated mixing inspector with register information, to make a check. The mixture inspection presenting step S66 presents mixture inspection medicine information on the medicine container 92, based upon the management information corresponding to the ID code of the medicine container 92.

The mixture inspection communicating step S67 communicates mixture inspection information, including mixing inspector information, to the dispensation managing section 11, while making the mixture inspection information correspond to the ID code of the medicine container 92.

In the medicine delivering section 18, the medicine delivering step S19 is performed. The medicine delivering step S19 has the delivery container identifying step S71, the delivery presenting step S72, the delivery person authenticating step S73, and the delivery communicating step S74.

The delivery container identifying step S71 obtains the ID code of the medicine container 92. The delivery presenting step S72 presents delivery permission/non-permission information on the medicine container 92, based upon the management information of the dispensation managing section 11 corresponding to the ID code of the medicine container 92.

The delivery person authenticating step S73 compares an authenticated delivery person with register information, to make a check. The delivery communicating step S74 communicates delivery information, including delivery person information and cart information, to the dispensation managing section 11, while making the delivery information correspond to the ID code of the medicine container. The progress inquiring step S81 and the progress responding step S19 are performed in the progress inquiring section 22.

The progress inquiring step S81 communicates dispensation inquiry information including the prescription code of the prescription direction to the dispensation managing section 11. The progress responding step S82 informs dispensation progress information corresponding to the prescription direction, based upon the management information of the ID code of the medicine container having the relation to the prescription code.

In the medicine withdrawing section 17, the withdrawal operation step S03 is performed. The withdrawal operation step S03 has the cancelling step S91, the container specifying step S92, the cancellation presenting step S93, the cancelled medicine determining step S94, the medicine withdrawing step S95, and the withdrawal communicating step S96.

The cancelling step S91 communicates it to the dispensation managing section 11, that the whole or part of the contents of the prescription direction has been canceled, based upon the prescription code of the prescription direction. The container specifying step S92 obtains the ID code of the medicine container 92 corresponding to the prescription code, based upon the management information of the dispensation managing section 11 including the prescription code. The cancellation presenting step S93 presents cancellation information on the medicine container 92.

The cancelled medicine determining step S94 determines whether or not a medicine in the medicine container 92 cancelled corresponds to a withdrawal medicine, based upon the management information and the cancellation information on the medicine container 92. The medicine withdrawing step S95 withdraws the cancelled medicine in the medicine container 92. The withdrawal communicating step S36 communicates withdrawal information, including a withdrawal medicine name and the number thereof, to the dispensation managing section 11, while making the information correspond to the ID code of the medicine container 92.

In the dispensation managing section 11, the management controlling step S86 and the updating step S87 are performed. The management controlling step S86 communicates with the medicine supplying section 14, the medicine procuring section 15, the medicine mixing section 16, the medicine delivering section 18, the progress inquiring section 22, and the medicine withdrawing section 17.

The updating step S87 updates such management information corresponding to the ID code of the medicine container as the prescription code, the dispensation direction information, the supply information, the procurement information, the procurement inspection information, the mixture information, the mixture inspection information, the delivery information, the withdrawal information and the like.

The medicine management system 12 performs the medicine management in the configuration as thus described.

It is to be noted that the medicine withdrawing operation may be performed in the medicine withdrawing section 17 as a place or a room different from the dispensation operating section. It is then possible to prevent a withdrawal medicine in the medicine container 92 cancelled from being intermingled into a medicine shelf or the like for unused medicines, and being lost or causing omission of the medicine withdrawing operation.

Further, the medicine withdrawing operation may be performed in the medicine withdrawing section 17 as a place, a room or the like which is different from the dispensation operating section. By immediately performing the medicine withdrawing operation in the dispensation operating section where the cancelled medicine container 92 is specified, it is possible to reduce the normal-temperature operation time as a normal-temperature stay time for the requiring cool medicine, so as to reduce disposal medicines.

### INDUSTRIAL APPLICABILITY

According to the medicine management method of the present invention, it is possible to accurately manage the dispensation operation, so as to perform an accurate withdrawal operation. Thereby, the method is useful in a hospital and the like equipped with dispensation operation facilities.

### REFERENCE MARKS IN THE DRAWINGS

- 11: Dispensation managing section
- 12: Medicine management system
- 13: Medical office
- 14: Medicine supplying section
- 15: Medicine procuring section
- 16: Medicine mixing section
- 17: Medicine withdrawing section
- 18: Medicine delivering section
- 20: Prescription display
- 21: Prescription generating section
- 22: Progress inquiring section
- 23: Prescription input device
- 24: Prescription communication device
- 25: Management communication device
- 26: Management storage device
- 27: Management processing device
- 31: Supply medicine storage device
- 32: Robot arm
- 33: Supply communication device
- 34, 41a, 51a, 65, 81a: RFID reader
- 35: Supply processing device
- 40: Medicine procurement shelf
- 41, 81: Medicine container placement section
- 42, 52, 62, 82: Person authentication device
- 43: Procurement display
- 44: Procurement communication device
- 45, 55, 88: Bar code reader
- 46: Procurement processing device
- 50: Medicine mixture shelf
- 51: Medicine container placement section
- 53: Mixture display
- 54: Mixture communication device
- 56: Mixture processing device
- 59, 89, 101: Button
- 63: Delivery display
- 64: Delivery communication device
- 66: Delivery shelf
- 67: Reset button
- 68: Delivery processing device
- 71: Inquiry input device
- 72: Inquiry display
- 73: Inquiry communication device
- 80: Medicine withdrawal shelf
- 83: Withdrawal display
- 84: Withdrawal communication device
- 85: Withdrawal processing device
- 91a, 91b: Belt conveyer
- 92: Medicine container
- 93: RFID tag
- 94, 96: Supply medicine storage device
- 95, 97: Medicine
- 98: Container correspondence display
- 99: Management medicine shelf case
- 100: Cool shelf case
- 107: Handle
- 111: Management medicine withdrawal case
- 112: Requiring cool medicine withdrawal case
- 120: Medicine management table
- 121: Withdrawal medicine
- 122: Label
- 123: Bar code
- 124: Cutoff line
- 130: Cancelled medicine management table

## Claims

1. A medicine management method, comprising:
a prescription generating step of generating a prescription direction which includes a prescription code and dispensation direction information, in a prescription generating section; and
a dispensation operation step of communicating with a dispensation managing section, by sending dispensation information regarding each medicine container dispensed based upon each prescription direction, while making the dispensation information correspond to said medicine container and the prescription code, and updating management information of said dispensation managing section, wherein
said method includes
a withdrawal operation step of specifying said medicine container that corresponds to the prescription code cancelled in accordance with the management information at a time of a cancellation of the prescription direction in said dispensation operation step, and directing a withdrawal process on said medicine container based upon cancellation information.

2. The medicine management method according to claim 1, wherein said withdrawal operation step has
a cancelled medicine determining step of determining whether or not a medicine in said medicine container cancelled corresponds to a withdrawal medicine, based upon the management information and the cancellation information on said medicine container.

3. The medicine management method according to claim 1, wherein said withdrawal operation step has
a cancellation presenting step of presenting the cancellation information on said medicine container.

4. The medicine management method according to claim 2, wherein said withdrawal operation step further has:
a medicine information detecting step of detecting medicine information on said medicine;
said cancelled medicine determining step of determining whether or not each detected medicine corresponds to a withdrawal medicine; and
a medicine withdrawal information presenting step of presenting a withdrawal medicine determination result of said each detected medicine as medicine withdrawal information.

5. The medicine management method according to claim 2, wherein said cancelled medicine determining step determines whether a medicine in said medicine container cancelled corresponds to a withdrawal medicine, corresponds to a disposal medicine, or corresponds to a non-cancelled medicine , based upon the management information and the cancellation information on said medicine container.

6. The medicine management method according to claim 2, wherein said cancelled medicine determining step determines whether or not a medicine in said medicine container cancelled corresponds to a withdrawal medicine, from dispensation progress information in the management information on said medicine container, the dispensation progress information indicating a dispensation progress status.

7. The medicine management method according to claim 6, wherein in a case of a mixture medicine being present in the medicines of said medicine container, said cancelled medicine determining step determines that said mixture medicine corresponds to a withdrawal medicine when the dispensation progress information in the management information on said medicine container indicates pre-mixture.

8. The medicine management method according to claim 6, wherein in a case of a requiring cool medicine that needs to be kept cool being present in the medicines of said medicine container, said cancelled medicine determining step calculates a normal-temperature operation time as a stay time for said requiring cool medicine after being taken out to a normal-temperature environment, from a medicine procurement time of the dispensation progress information in the management information on said medicine container, and determines that the requiring cool medicine corresponds to a withdrawal medicine when the normal-temperature operation time is shorter than a normal-temperature management time.

9. The medicine management method according to claim 5, wherein said withdrawal operation step further has
a cancellation authentication recording step of recording cancellation authentication information indicating a cancelled medicine to a withdrawal medicine or a disposal medicine determined in said cancelled medicine determining step.

10. The medicine management method according to claim 9, wherein
a label recorded with the cancellation authentication information is issued in said cancellation authentication recording step.

11. The medicine management method according to claim 2, further comprising
a return operation step of returning a medicine determined as a withdrawal medicine in said withdrawal operation step to a dispensation operating section in which said dispensation operation is performed, wherein
said return operation step has
a return person authenticating step of obtaining operator information of a return person, and returning said medicine to said dispensation operating section when said return person is qualified to return said medicine.

12. The medicine management method according to claim 9, further comprising
a return operation step of returning a medicine determined as a withdrawal medicine in said withdrawal operation step to said dispensation operating section in which the dispensation operation is performed, wherein
said cancellation authentication recording step records the cancellation authentication information including the cancelled medicine code of said withdrawal medicine, while recording the cancelled medicine code into said dispensation managing section, and
said return operation step includes a cancelled medicine checking step of determining whether or not the cancelled medicine code in the cancellation authentication recording information, detected in said dispensation operating section, is recorded in said dispensation managing section, at a time of returning said medicine determined as a withdrawal medicine to said dispensation operating section.

13. The medicine management method according to claim 1, wherein
said dispensation operation step further has:
a medicine supplying step of automatically supplying medicines in a medicine supplying section;
a medicine procuring step of procuring a medicine in a medicine procuring section;
a procurement inspecting step of inspecting the procurement in said medicine procuring section;
a medicine mixing step of mixing medicines in a medicine mixing section; and
a mixture inspecting step of inspecting the mixture in said medicine mixing section,
said medicine supplying step has:
an automatic supply step of automatically supplying medicines into said medicine container in accordance with the prescription direction; and
a supply communicating step of making the ID code of said medicine container and the prescription code of the prescription direction have a corresponding relation to each other, and communicating the corresponding relation and supply information regarding said medicines inserted into said medicine container to said dispensation managing section,
said medicine procuring step has:
a procurement person authenticating step of authenticating a procurement person and making a check by comparing with register information;
a procurement container identifying step of identifying the ID code of said medicine container;
a procurement presenting step of presenting supplementary medicine information on said medicine container, based upon the management information of said dispensation managing section corresponding to the ID code of said medicine container; and
a procurement communicating step of communicating procurement information, including information of said procurement person, to said dispensation managing section, while making the procurement information correspond to the ID code of said medicine container,
said procurement inspecting step has:
a procurement inspector authenticating step of authenticating a procurement inspector and making a check by comparing with register information in said medicine procuring section;
a procurement inspection presenting step of presenting information of a procurement medicine procured in said medicine container corresponding to the ID code of said medicine container; and
a procurement inspection communicating step of communicating procurement inspection information, including procurement inspector information, to said dispensation managing section, while making the procurement inspection information correspond to the ID code of said medicine container,
said medicine mixing step has:
a mixing person authenticating step of authenticating a mixing person and making a check by comparing with register information;
a mixture container identifying step of obtaining the ID code of said medicine container;
a mixture presenting step of presenting mixture medicine information of said medicine container based upon the management information of said dispensation managing section corresponding to the ID code of the medicine container; and
a mixture communicating step of communicating mixture information, including mixing person information, to said dispensation managing section, while making the mixture information correspond to the ID code of said medicine container, and
said mixture inspecting step has:
a mixing inspector authenticating step of authenticating a mixing inspector and making a check by comparing with register information;
a mixture inspection presenting step of presenting mixture inspection medicine information of mixture medicines in said medicine container based upon the management information of said dispensation managing section corresponding to the ID code of said medicine container; and
a mixture inspection communicating step of communicating mixture inspection information, including mixing inspector information, to said dispensation managing section, while making the mixture inspection information correspond to the ID code of said medicine container.
